# EUROPEAN PATENT SPECIFICATION

(11) **EP 3 478 233 B1**
(45) Date of publication and mention of the grant of the patent: **09.06.2021**
(21) Application number: 17820700.7
(22) Date of filing: 14.04.2017
(51) Int. Cl.: A61F 11/00, A61M 3/02

(54) **WEARABLE EAR CLEANING DEVICE**
AM KÖRPER TRAGBARER OHRREINIGER
DISPOSITIF DE NETTOYAGE DES OREILLES PORTABLE

(30) Priority: 30.06.2016 US 201662357320 P
(43) Date of publication of application: 08.05.2019
(73) Proprietor: Safkan, Inc., Renton, Washington 98058 (US)
(72) Inventor: DIWAN, Aadil, Renton Washington 98058 (US)
(74) Representative: Cooley (UK) LLP
(86) International application number: PCT/US2017/027763
(87) International publication number: WO 2018/004785

(56) References cited:
- EP-A2- 0 099 759
- CH-A2- 702 267
- US-A- 5 364 343
- US-A1- 2002 173 746
- US-A1- 2011 015 489
- US-B1- 7 699 820
- US-B1- 8 328 830
- US-B2- 8 603 152

## Description

### TECHNICAL FIELD

The present disclosure generally relates to providing a wearable ear cleaning device.

### BACKGROUND

### Description of the Related Art

Ear wax, also known as cerumen, naturally forms in the outer portion of a person's auditory canal, and serves to protect and lubricate the auditory canal. The motion of a person's jaw assists in moving old ear wax towards the outside of the auditory canal where it dries up and falls away. Unfortunately, ear wax may build up in the auditory canal to the point that it impacts a person's hearing. Moreover, some people attempt to clean their ears with devices, such as cotton swabs, that generally push ear wax deeper into the auditory canal towards the ear drum. For this reason, attempting to clean the auditory canal with a cotton swab may actually harm a person's hearing without providing any substantial benefit.

CH702267 discloses a device has an injecting unit for injecting cleaning solution into an ear canal and for sucking the cleaning solution and dissolved contaminatior The device employs a separate spray nozzle and suction nozzle.

In EP0099759, binaural thermal-response differentials which are symptomatic of vertigo, particularly Meniere's disease, are treated by establishing a thermal treatment operative via liquid contact with at least one eardrum, thereby establishing a difference in thermal exposure of one ear with respect to the other.

US5,364,343 discloses an irrigation device for use in an ear canal. A main body of substantially conical shape has a central axis. There is at least one input duct connecting to a lavage liquid delivery apparatus for injecting the lavage liquid into the ear canal. There is at least one output duct allowing the continuous drainage of used lavage liquid.

### BRIEF SUMMARY

In accordance with one aspect of the invention, there is provided a device to irrigate ear canals of human ears as defined by claim 1. Optional features are defined by the dependent claims.

To clean the auditory canal, a mixture of saline, hydrogen peroxide, and/or water is used to irrigate the auditory canal and remove an excessive build-up of ear wax. Irrigating the auditory canal, however, may require a visit to a physician and the use of devices that require multiple people to operate. In addition, devices and techniques that are used to irrigate the auditory canal may result in used cleaning agent or cleaning fluid, usually carrying flushed ear wax, to exit the auditory canal and impact the patient.

A device to irrigate ear canals of human ears may be summarized as including a first over-ear earpiece sized and dimensioned to be worn over human ears and having a cavity sized and dimensioned to receive one human ear, the first over-ear earpiece comprising: a first cleaning agent reservoir to hold cleaning agents; a first discharge collection reservoir to collect discharge from irrigation; a first cannula coupler interface to which cannulas are selectively detachably physically coupleable; a first cleaning fluid fluidly communicative path that provides fluid communication between the first cleaning agent reservoir and the first cannula coupler interface; and a first discharge fluidly communicative path that provides fluid communication between the first discharge collection reservoir and the first cannula coupler interface.

The device may further include a second over-ear earpiece sized and dimensioned to be worn over human ears and having a cavity sized and dimensioned to receive one human ear, the second over-ear earpiece comprising: a second cleaning agent reservoir to hold cleaning agents; a second discharge collection reservoir to collect discharge from irrigation; a second cannula coupler interface to which cannulas are selectively detachably physically coupleable; a second cleaning fluid fluidly communicative path that provides fluid communication between the second cleaning agent reservoir and the second cannula coupler interface; and a second discharge fluidly communicative path that provides fluid communication between the second discharge collection reservoir and the second cannula coupler interface.

The device may further include an adjustable head strap that adjustably connects the first over-ear earpiece and the second over-ear earpiece in spaced apart relation from one another, the adjustable head strap sized and dimensioned to be worn on a human head with the first over-ear earpiece positioned over a first ear and the second over-ear earpiece positioned over a second ear.

The device may further include at least a first pivot coupler that pivotally couples the first over-ear earpiece to the adjustable head strap to pivot about a first axis; and at least a second pivot coupler that pivotally couples the second over-ear earpiece to the adjustable head strap to pivot about a second axis, the first and the second axes perpendicular to an axis that extends between the respective cannula coupler interfaces of the first and the second over-ear earpieces.

The first cleaning agent reservoir may have a port, the first discharge collection reservoir may have a port, and the first over-ear earpiece may further include a first housing, the first housing comprises a pan and a lid which form an interior of the first housing, with a first passage that extends through the first housing and which is aligned with and sized and dimensioned to mate with the port of the first cleaning agent reservoir and a second passage that extends through the first housing and which is aligned with and sized and dimensioned to mate with the port of the first discharge collection reservoir, the interior of the first housing sealed from the first and the second passages.

The first over-ear earpiece may further include a first annular bracket, the first annular bracket pivotally coupled to the adjustable head strap, and the first housing selectively detachably coupled to the first annular bracket.

The first over-ear earpiece may further include a resilient or conformable annular membrane which at least partially forms the cavity sized and dimensioned to receive one ear.

The first over-ear earpiece may further include a first shell that forms the first cleaning agent reservoir and a second shell that forms the first discharge collection reservoir. The first discharge collection reservoir may be selectively detachable with respect to the first cleaning agent reservoir. The first cleaning agent reservoir may be selectively detachable with respect to the first discharge collection reservoir.

The first over-ear earpiece may further include a first cover that is detachable securable to the second shell and which forms the first discharge collection reservoir along with the second shell.

The first over-ear earpiece may further include a second cover that is detachably securable to the first shell and which forms the first cleaning agent reservoir along with the first shell.

The first over-ear earpiece may further include a first housing to which the first discharge collection reservoir and the first cleaning agent reservoir are selectively detachable.

The first over-ear earpiece may further include a first pair of ferromagnetic couplers that detachably magnetically couple the first cleaning reservoir to the first housing.

The first over-ear earpiece may further include a second pair of ferromagnetic couplers that detachably magnetically couple the first discharge collection reservoir to the first housing.

The first over-ear earpiece may further include at least one pump fluidly coupled to move cleaning agent along the first cleaning fluid fluidly communicative path from the cleaning agent reservoir toward the first cannula coupler interface.

The first over-ear earpiece may further include at least one pump fluidly coupled to apply a negative pressure to move discharge along the first discharge fluidly communicative path from the first cannula coupler interface toward the first discharge collection reservoir.

The first over-ear earpiece may further include a first housing, at least one pump fluidly coupled to at least one of the first cleaning fluid fluidly communicative path or the discharge fluidly communicative path, and a first set of controller circuitry housed in the first housing and communicatively coupled to control the at least one pump.

The first over-ear earpiece may further include a number of user-actable selectable controls accessible from an exterior of the first over-ear earpiece, the user-actable selectable controls communicatively coupled to the first set of controller circuitry communicatively coupled to control the at least one pump.

The first over-ear earpiece includes a cannula having a proximate end and a distal end, an irrigation inlet port positioned at te proximate end, a discharge collection outlet port positioned at the proximate end, a plurality of irrigation outlet apertures positioned relatively toward the distal end with respect to the irrigation inlet port, the irrigation outlet apertures in fluid communication with the irrigation inlet port, and a discharge collection inlet port positioned relatively toward the distal end with respect to the discharge collection outlet port, the discharge collection inlet port in fluid communication with the discharge collection outlet port.

The disposable cannula includes a flow path that extends betwen the discharge collection inlet port and the discharge collection outlet port and a trap positioned in the flow path between the discharge collection inlet port and the discharge collection outlet port.

The device may further include a first processor, and a first nontransitory computer-readable medium communicatively coupled to the first processor, wherein the first nontransitory computer-readable medium stores first processor-executable instructions that specifically program the first processor to: provide a number of signals to at least one pump that causes the at least one pump to dispense a first quantity of cleaning fluid along the first cleaning fluid fluidly communicative path from the first cleaning agent reservoir and to produce a negative pressure along at least a portion of the first discharge fluidly communicative path. The first processor-executable instructions may specifically program the first processor to time-delay the production of the negative pressure from the dispensation of the first quantity of cleaning fluid for a first period of time. The first processor-executable instructions may specifically program the first processor to time-delay the production of the negative pressure from the dispensation of the first quantity of cleaning fluid for at least one minute.

A cannula may be summarized as including a body, the body having a length, a proximal end, and a distal end, the distal end opposite the proximal end across the length of the body, the body additionally having an irrigation inlet port positioned at the proximal end, a discharge collection outlet port positioned at the proximal end, a plurality of irrigation outlet apertures positioned relatively toward the distal end with respect to the irrigation inlet port, and a discharge collection inlet port positioned at the distal end, the body further having at least one irrigation passage that provides at least one irrigation flow path between the irrigation inlet port and the plurality of irrigation outlet apertures, at least one discharge collection passage that provides at least one discharge flow path that extends between the discharge collection inlet port and the discharge collection outlet port, and at least one trap positioned in the discharge passage between the discharge collection inlet port and the discharge collection outlet port in the at least one discharge flow path.

The body may further include an interface sized and dimensioned to removably physically couple to a complementary interface of an over-ear earpiece and to align the irrigation inlet port of the cannula with a cleaning agent port of the over-ear earpiece and to concurrently align the discharge collection outlet port with a vacuum port of the over-ear earpiece when the cannula is physically coupled to the over-ear earpiece. The at least one trap may be a filter that extends at least partially across the discharge collection passage. The discharge collection passage may have an inside perimeter, wherein the at least one trap may include a plurality of projections that extend radially inward from the inside perimeter of the discharge collection passage towards a central axis of the discharge collection passage. The at least one trap may be sized and dimensioned to trap physical debris of at least one defined dimension while passing at least one of a quantity of a liquid and air. The plurality of irrigation outlet apertures may include at least three irrigation outlet apertures proximate the distal end. At least one of the at least three irrigation outlet apertures may be sized and shaped to direct a flow of cleaning agent that exits the at least one of the at least three irrigation outlet apertures radially outward from the distal end of the cannula. The proximal end has a respective cross-sectional area, the distal end has a respective cross-sectional area and the cross-sectional area of the distal end is less than the cross-sectional area of the proximal end, and the body of the cannula tapers from the proximal end towards the distal end. The tapered body is sized and shaped such that as the cannula is inserted into a human auditory canal that has a side wall and leads to an ear drum, a portion of the body of the cannula impacts the side wall of the human auditory canal before the distal end of the cannula impacts the ear drum. The body of the cannula may form a frustro-conical shape and the distal end may include a beveled portion. The proximal end may include an interior wall with an outside diameter and an exterior wall with an interior diameter, and the interior wall and the exterior wall may be sized and shaped to removably securely engage a complementary interface of an over-ear earpiece between the outside diameter of the interior wall and the interior diameter of the exterior wall. The body may be a unitary single-piece plastic body and may have a form that is a body of rotation. The irrigation inlet port may be radially offset from the discharge collection outlet port. The body may have a central/longitudinal axis and the discharge collection inlet port may be disposed about the central/ longitudinal axis. The body may have a central/longitudinal axis and the irrigation outlet apertures may be radially offset outwardly from the central/ longitudinal axis.

### BRIEF DESCRIPTION OF THE SEVERAL VIEWS OF THE DRAWINGS

In the drawings, identical reference numbers identify similar elements or acts. The sizes and relative positions of elements in the drawings are not necessarily drawn to scale. For example, the shapes of various elements and angles are not necessarily drawn to scale, and some of these elements are arbitrarily enlarged and positioned to improve drawing legibility. Further, the particular shapes of the elements as drawn, are not necessarily intended to convey any information regarding the actual shape of the particular elements, and have been solely selected for ease of recognition in the drawings.
Figure 1A is an isometric view of an irrigation device, according to at least one illustrated implementation.
Figure 1B is an isometric view of an over-ear earpiece, according to at least one illustrated implementation.
Figure 2A is an exploded view of one of the over-ear earpieces of an irrigation device, according to at least one illustrated implementation.
Figure 2B is an isometric view of an over-ear earpiece that has a rectangular form factor, according to at least one illustrated implementation.
Figures 3A is an isometric view of a first side of a pan that may be included as a component of an over-ear earpiece, according to at least one illustrated implementation.
Figures 3B is an isometric view of a second side of the pan of Figure 3A, according to at least one illustrated implementation.
Figures 4 is an isometric view of a lid that may be included as a component of an over-ear earpiece, according to at least one illustrated implementation.
Figures 5 is an isometric view of a cleaning agent reservoir of an over-ear earpiece, according to at least one illustrated implementation.
Figures 6 is an isometric view of a discharge reservoir of an over-ear earpiece, according to at least one illustrated implementation.
Figure 7A is an isometric view of a disposable cleaning agent reservoir of an over-ear earpiece, according to at least one illustrated implementation.
Figure 7B is an isometric view of a disposable discharge collection reservoir of an over-ear earpiece, according to at least one illustrated implementation.
Figure 7C is an isometric view of a shell broken into two cavities, one of which is sized and dimensioned to hold a disposable cleaning agent reservoir and the other of which is sized and dimensioned to hold a disposable discharge collection reservoir, according to at least one illustrated implementation.
Figure 7D is an isometric view of a unitary disposable container that includes separate sections for a disposable cleaning agent reservoir and a disposable discharge collection reservoir, according to at least one illustrated implementation.
Figure 8 is a side plan view of a cannula, according to one implementation, which may be used with or as part of an irrigation device.
Figure 9 is a front, side isometric view of the cannula of Figure 7, showing the tip from which cleaning agent exits and discharge enters the cannula.
Figure 10 is a rear, side isometric view of the cannula of Figure 7.
Figure 11 is a rear plan view of the cannula of Figure 7, which includes an interface that is used to couple the cannula to a cannula coupler interface on the over-ear earpieces.
Figure 12 is a transparent side, rear isometric view of the cannula of Figure 7, that shows the paths of an irrigation passage and a discharge collection passage within the interior of the cannula.
Figure 13 is a block diagram of controller circuitry and a power supply, according to one illustrated implementation, which may be used with or as part of an irrigation device.
Figure 14 shows an example of user-actable selectable controls, according to at least one illustrated implementation.
Figure 15 provides a flow diagram of a set of user-actable selectable controls for operating an irrigation device, according to at least one illustrated implementation.

### DETAILED DESCRIPTION

In the following description, certain specific details are set forth in order to provide a thorough understanding of various disclosed implementations. However, one skilled in the relevant art will recognize that implementations may be practiced without one or more of these specific details, or with other methods, components, materials, etc. In other instances, structures and solutions associated with ear cleaning, including the various components and ratios of such components in cleaning solutions, have not been shown or described in detail to avoid unnecessarily obscuring descriptions of the implementations.

Unless the context requires otherwise, throughout the specification and claims which follow, the word "comprise" and variations thereof, such as "comprises" and "comprising," are to be construed in an open, inclusive sense, that is, as "including, but not limited to."

Reference throughout this specification to "one implementation" or "an implementation" means that a particular feature, structure or characteristic described in connection with the implementation or implementations is included in at least one implementation or implementations. Thus, the appearances of the phrases "in one implementation" or "in an implementation" in various places throughout this specification are not necessarily all referring to the same implementation. Furthermore, the particular features, structures, or characteristics may be combined in any suitable manner in one or more implementation or implementations.

As used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

The headings and Abstract of the Disclosure provided herein are for convenience only and do not interpret the scope or meaning of the implementations.

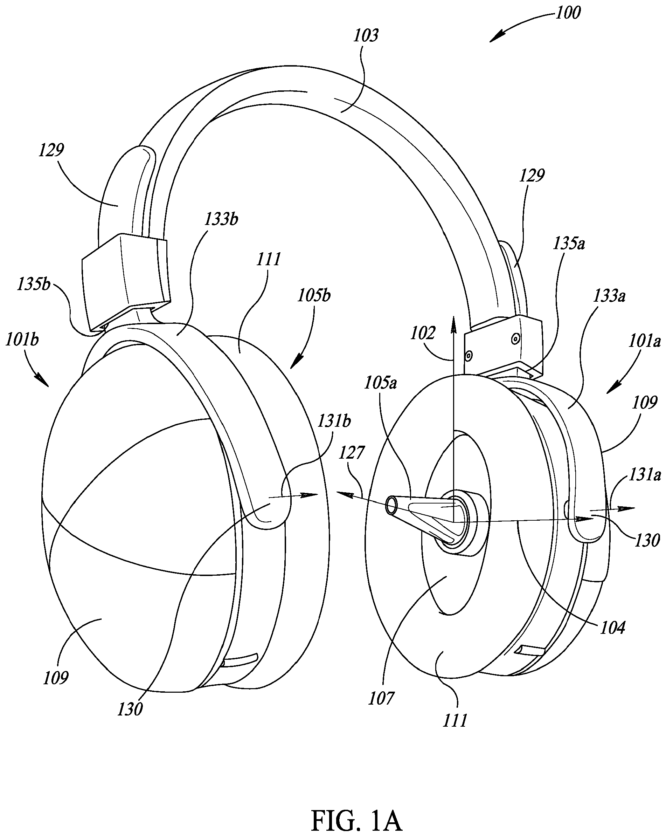

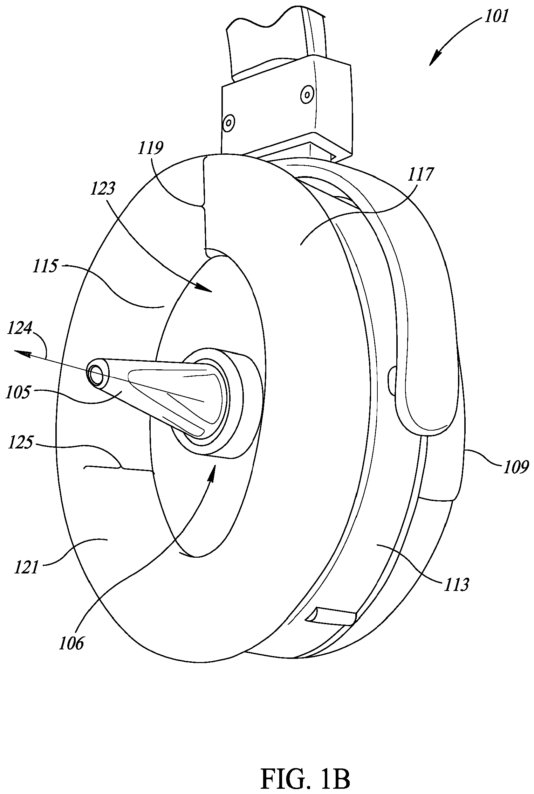

Figure 1A shows an irrigation device 100 that can be worn on a head of a human, according to at least one illustrated implementation. The irrigation device 100 includes a first and a second over-ear earpiece 101 a, 101 b (generically, "over-ear earpiece 101"), an adjustable head strap 103, and a first and a second cannula 105a, 105b (generically, "cannula 105") connected to respective cannula coupler interfaces 106a, 106b (only one visible in Figure 1A). Figure 1B shows the over-ear earpiece 101 in more detail.

In some implementations, the irrigation device 100 may be sized and shaped such that the first over-ear earpiece 101a fits over a user's right ear, and the second over-ear earpiece 101 b fits over the user's left ear. Each over-ear earpiece may have a vertical axis 102 that is substantially perpendicular to the ground when a user wearing the irrigation device 100 is in an upright position, and a horizontal axis 104 that that is substantially parallel to the ground when the user wearing the irrigation device 100 is in the upright position is. While discussed with respect to an upright positon for ease of reference, it is noted that a user does not necessarily need to be in an upright position during use of the irrigation device 100.

Each over-ear earpiece 101 may include an interior side 107 and an opposing exterior side 109 in which the interior side 107 is directed towards and is adjacent the side of the user's head when the user wears the irrigation device 100. Each over-ear earpiece 101 may have a perimeter 113 that extends between the interior side 107 and the exterior side 109. The perimeter 113 may be sized and shaped so that the over-ear earpiece 101 can enclose a range of ear shapes. In some implementations, the perimeter 113 of the over-ear earpiece 101 may be substantially circular, oval, and/or elliptical in shape. In some implementations, the over-ear earpiece 101 may include a port 114 that may be used to provide power and/or communications to the irrigation device 100. The charging may be accomplished, for example, by connecting one or more physical connectors (*e.g*., a USB connector) to thereby charge in internal battery within the irrigation device. In some implementations, the port 114 may enable inductive coupling to charge an internal battery. In some implementations, only one of the over-ear earpieces 101 may include a port 114. In some implementations, each of the over-ear earpieces 101 may be electrically coupled via, for example, one or more electrical connections running through the adjustable head strap 103.

The over-ear earpiece 101 may include an annular membrane 111 positioned on the interior side 107 at or proximate the perimeter 113 of the over-ear earpiece 101. In some implementations, the annular membrane 111 has an interior wall 115 and an exterior wall 117 that are separated by a distance 119 such that the interior wall 115 is concentric with, and completely enclosed by, the exterior wall 117. The interior wall 115 and the exterior wall 117 may be connected by a rim 121 that traverses the distance 119. In some implementations, the rim 121 may be a surface that curves outward, away from the over-ear earpiece 101, such as shown in Figure 1. In some implementations, the annular membrane 111 may be sized and shaped to resemble a portion of a solid torus (*e.g*., the top half of a doughnut). Such a curved or partially toroidal surface may be used to increase the user's comfort when wearing the over-ear earpiece 101. In some implementations, the rim 121 may form a substantially flat surface that is perpendicular to one or both of the interior wall 115 and the exterior wall 117.

In some implementations, the annular membrane 111 may be made of cushioned material that compresses when a force is applied against it. Such material may include, for example, an elastomer, closed-cell foam (e.g., polyurethane), open cell foam, gel in a pouch, silicone, and/or rubber which may include an outer cover of plastic, leather, or leatherette material. In some implementations, the annular membrane 111 may be comprised of resilient material to increase its longevity and durability. Such cushioned and/or resilient material may be used to provide a more comfortable fit for the user when wearing the irrigation device 100. In some implementations, the cushioned material may conform to the shape of the side of the user's head when the irrigation device 100 is in use. As such, the cushioned material may thereby form a seal against the user's head to trap cleaning agent that escapes from the cannula 105 and/or the user's auditory canal during an irrigation procedure. The annular membrane 111 may be comprised, for example, of elastomer-based materials. In some implementations, the annular membrane 111 may be formed of a core composed at least in part of elastomer-based materials and a corresponding cover that may be composed of material that protects the core from the surrounding environment, such as a plastic or other synthetic material that does not degrade when wet. In some implementations, the annular membrane 111 may be detachably removed from the over-ear earpiece 101, and thus may be replaced after each use of the irrigation device 100.

The interior wall 115 of the annular membrane 111 forms a cavity 123 that is sized and shaped to enclose and receive one of the user's ears. The size and shape of the annular membrane 111 and the cavity 123 may further be used to position the cannula 105 within the user's auditory canal when the user is wearing the irrigation device 100. In some implementations, the cavity 123 may be cylindrical in shape and have the cannula 105 oriented along a central axis 124 of the cavity 123. In some implementations, the cavity 123 may be elongated such that the perimeter of the interior wall 115 forms a substantially elliptical or oval shape. In such an implementation, the cavity may be relatively longer along the vertical axis 102, and relatively shorter along the horizontal axis 104. Elongating the cavity 123 in such a way may provide for a better, more comfortable fit for the user. The cavity 123 may further be sized and dimensioned to receive ears of various sizes.

The cavity 123 may have a depth formed by a height 125 of the interior wall 115. In some implementations, the depth of the cavity 123 may be less than a height of the cannula 105, enabling the cannula 105 to extend past the rim 121 of the annular membrane 111 and thereby enter the user's auditory canal when the user wears the irrigation device 100. In some implementations, as discussed below, the cannula 105 may further be tapered, with a tip that is narrower than a base of the cannula 105, to assist in inserting and positioning the tip of the cannula 105 into the user's auditory canal. The tapering of the cannula 105, however, can be designed such that a portion of the cannula 105 between the tip and the base impacts the outside opening of the user's auditory canal before the tip of the cannula 105 impacts the user's ear drum, thereby preventing injury to the ear drum.

In some implementations, the irrigation device 100 includes an adjustable head strap 103 that adjustably connects the first over-ear earpiece 101a and the second over-ear earpiece 101b in a spaced apart relation along a lateral axis 127 that extends between the two opposing cannula coupler interfaces 106a and 106b. The adjustable head strap 103 may be sized and shaped to fit over the user's head, thereby concurrently positioning the first over-ear earpiece 101 a over a user's first ear and the second over-ear earpiece 101b over the user's second ear. The adjustable head strap 103 may be flexible such that the first over-ear earpiece 101 a and the second over-ear earpiece 101b may be moved or flexed laterally along the lateral axis 127. In some implementations, one or both of the over-ear earpieces 101a, 101b may include vertical adjustors 129 that allow the over-ear earpieces 101 to move along the vertical axis 102 with respect to the adjustable head strap 103 when a user is wearing the irrigation device 100. The vertical adjustors 129 may be used, for example, to adjust the irrigation device to accommodate different circumferential distances between various users' ears (e.g., from one ear of a user, over the top of the user's head, and to the other ear of the user). Thus, one or both of the vertical adjustors 129 may be extended to accommodate users having relatively larger heads.

The adjustable head strap 103 may connect to each of the over-ear earpieces 101 via one or more pivot couplers 130. The pivot couplers 130 may enable each of the over-ear earpieces 101a, 101b to independently pivot around respective axes 131a, 131b that run between the pivot couplers 130 on each respective over-ear earpieces 101 a, 101b; the respective axes 131a, 131b may be perpendicular to the lateral axis 127 that extends between the first cannula coupler interface 106a and the second cannula coupler interface 106b. Such pivot couplers 130 enable each of the over-ear earpieces 101 a, 101b to more comfortably fit the contours and shape of a user's head when the user operates the irrigation device 100.

In some implementations, the over-ear earpieces 101a, 101b may include respective annular brackets 133a and 133b. The annular brackets 133a, 133b may be sized and shaped to be slightly larger than a portion of the perimeter 113 of the respective over-ear earpiece 101 a, 101b. For example, the annular brackets 133a, 133b may be sized and shaped to run along the top half of the perimeters 113 of the respective over-ear earpieces 101 a, 101b. In such implementations, the annular brackets 133a, 133b may be pivotally coupled to the adjustable head strap 103 at junctions 135a and 135b, thus enabling each of the over-ear earpieces 101 to independently pivot relative to the junctions 135a and 135b.

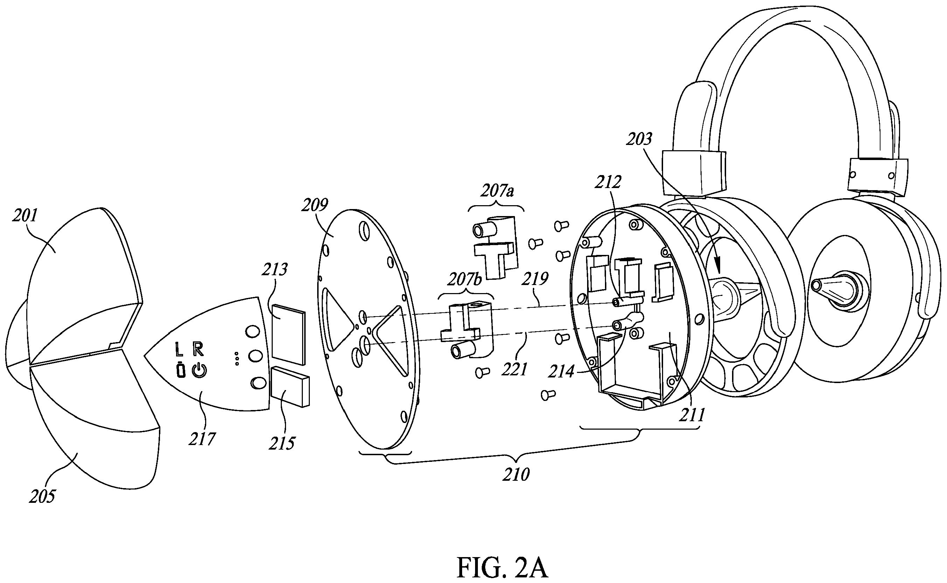

Figure 2A shows the components of one of the over-ear earpieces 101 of the irrigation device 100, according to at least one illustrated implementation. The components of the over-ear earpiece 101 may include a cleaning agent reservoir 201, a cannula coupler interface 203, and a discharge collection reservoir 205. Components of the over-ear earpiece 101 may also include a pump assembly 207a that may have a pressure pump and/or manifold to couple to the cleaning agent reservoir 201 and operable to generate a positive pressure (*e.g*., greater than one atmosphere or greater than ambient pressure) and a vacuum assembly 207b that may have a vacuum pump and/or manifold to couple to the discharge collection reservoir 205 and operable to generate a negative pressure (*e.g*., less than one atmosphere or less than ambient pressure), a housing 210 that includes a lid 209 and a pan 211, controller circuitry 213, one or more power supplies 215, and a set of user-actable selectable controls 217.

The cleaning agent reservoir 201 holds cleaning agents that are to be used to clean the user's auditory canal. Such a cleaning agent may include, for example, one or more of saline, hydrogen peroxide, and/or water. In some implementations, the cleaning agent reservoir 201 may be filled with cleaning agent that is heated to a temperature at, or slightly above or below, the user's body temperature for the user's comfort. In some implementations, the cleaning agent may be reservoir 201 may be filled with cleaning agent at or around body temperature or slightly warmer. In some implementations, the cleaning agent reservoir 201 may include or be physically coupled to a heating component (*e.g*., heater or resistive element) positioned to heat the contained cleaning agent to and/or maintain the contained cleaning agent at a temperature at or around the user's body temperature. Such a heating component may be electrically and communicatively coupled to the controller circuitry 213, which may transmit control signals to the heating component. In some implementations, the cleaning agent reservoir 201 may include an internal thermometer to measure the temperature of the cleaning agent contained within the cleaning agent reservoir 201. In such an implementation, the thermometer may by electrically and communicatively coupled to the controller circuitry 213. In such an implementation, the thermometer may transmit to the controller circuitry 213 electrical signals indicative of the temperature within the cleaning agent reservoir 201. In some implementations, the controller circuitry 213 may use the signals from the thermometer as feedback to control the heating component. In some implementations, the controller circuitry 213 may use the signals from the thermometer to provide for the safety and/or comfort of the user by preventing an irrigation procedure from being performed when the cleaning agent held within the cleaning agent reservoir 201 is either too hot or too cold.

The cleaning agent reservoir 201 is in fluid communication with the cannua coupler interface 203 using a cleaning fluid fluidly communicative path 219. As shown in Figure 2A, the cleaning fluid fluidly communicative path 219 may proceed through an opening in the lid 209, through a cleaning agent passage 212 in the housing 210, to the cannula coupler interface 203. When an irrigation procedure begins, cleaning agent exits the cleaning agent reservoir 201 and proceeds through the cleaning fluid fluidly communicative path 219 to the cannula 105. Air or liquid pressure from the pump assembly 207a may be applied to the cleaning agent reservoir 201 to facilitate the flow of the cleaning agent from the cleaning agent reservoir 201. In some implementations, the pump assembly 207a may be a water or liquid pump that is used to draw the cleaning agent from the cleaning agent reservoir 201 and discharge the cleaning agent towards the cannula 105 at a desired positive pressure. The cleaning agent may proceed into the cannula to be discharged through one or more irrigation outlet apertures, as discussed further below.

In some implementations, the irrigation device 100 may leave the cleaning agent in the user's auditory canal for a period of time (e.g., at least one minute) before removing the cleaning agent. In some implementations, this time period may be set by the user via the user-actable selectable controls 217. In some implementations, the controller circuitry 213 may store processor-executable instructions that define one or more pre-set time periods for the cleaning agent to remain in the user's auditory canal. The user may choose one of these pre-set time periods via the user-actable selectable controls 217 such that the associated processor-executable instructions are executed by a processor included in the controller circuitry 213. This time period between introducing the cleaning agent into and removing it from the auditory canal may enable the cleaning agent to further soften buildup in the user's auditory canal and thus enhance the cleaning capabilities of the irrigation device 100 and the user's experience.

When the time period ends, the discharge from the irrigation is removed from the user's auditory canal via a discharge collection inlet port located on the cannula 105, as discussed below. The discharge exits the cannula 105 to a discharge fluidly communicative path 221 that proceeds from the cannula coupler interface 203, through a discharge passage 214 in the housing 210, and through one or more openings in the lid 209, to the discharge collection reservoir 205. In some implementations, the vacuum assembly or vacuum pump 207b is used to create a vacuum or area of low pressure (i.e., lower than ambient environmental pressure, e.g., lower than 1 atmosphere) within the discharge collection reservoir 205 to facilitate the removal of the discharge from the auditory canal. References to vacuum herein and in the claims refer to a pressure that is lower than ambient environmental pressure (e.g., lower than 1 atmosphere) rather than an absolute vacuum.

In some implementations, the irrigation device 100 may pulse cleaning agent into the user's auditory canal for a period of time (*e.g.*, up to 30 seconds, 35 seconds, up to a one minute, or longer). Such pulses may occur, for example, every couple of seconds. In such an implementation, the vacuum assembly 207b may be actuated before the cleaning agent is first pulsed into the user's auditory canal and remain actuated during the entire time period. The vacuum assembly 207b may be deactivated after the time period ends.

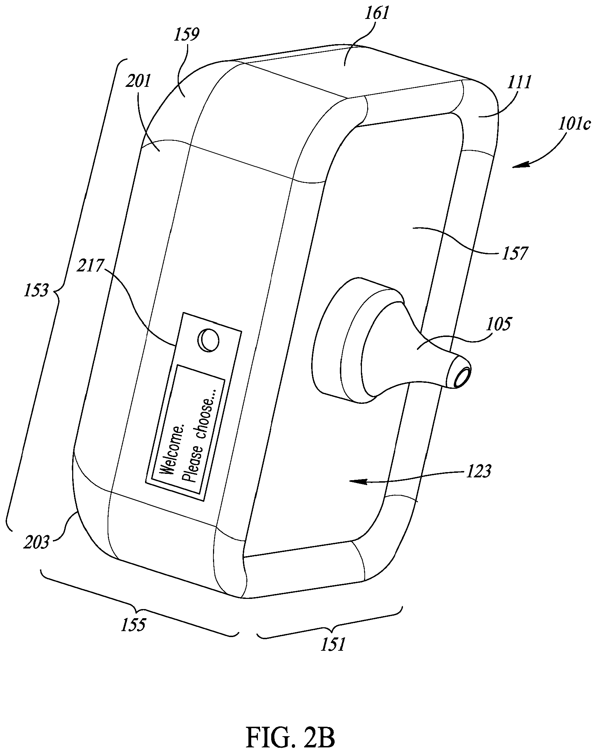

Figure 2B shows an implementation of an irrigation device 100 with a rectangular over-ear earpiece 101c, according to one illustrated implementation. Such a rectangular over-ear earpiece 101c may have a width 151, a height 153, and a depth 155 with an interior side 157 and an exterior side 159 that are connected by one or more side walls 161. In some implementations, the depth 155 may be less than the width 151, and the width 151 may be less than the height 153. Such dimensions may provide a more streamlined fit and appearance when compared to over-ear earpieces 101 of other shapes. When placed over an ear of a user, the height 153 may be oriented vertically with the interior side 157 adjacent to the side of the face of the user, perpendicular to the ground when the user wearing the irrigation device 100 is in an upright position. The cannula 105 may be located substantially in the center of the interior side 157 of the rectangular over-ear earpiece 101c to position the cannula 105 within the auditory canal of the user when the user is wearing the irrigation device 100 with the rectangular over-ear earpiece 101c. The rectangular over-ear earpiece 101c may include an annular membrane 111 that extends around the perimeter of the interior side 157. As discussed above, the annular membrane may form a cavity 123 that may be sized and dimensioned to receive ears of various sizes. The cleaning agent reservoir 201 and a discharge collection reservoir 205 may be enclosed within one or more containers attached to the rectangular over-ear earpiece 101c along the exterior side 159. A set of set of user-actable selectable controls 217 may be located along one of the side walls 161. In some implementations, the set of user-actable selectable controls 217 may include one or more buttons, switches, and/or a touch screen pad for entering commands to, and receiving status updates and feedback from, the irrigation device 100.

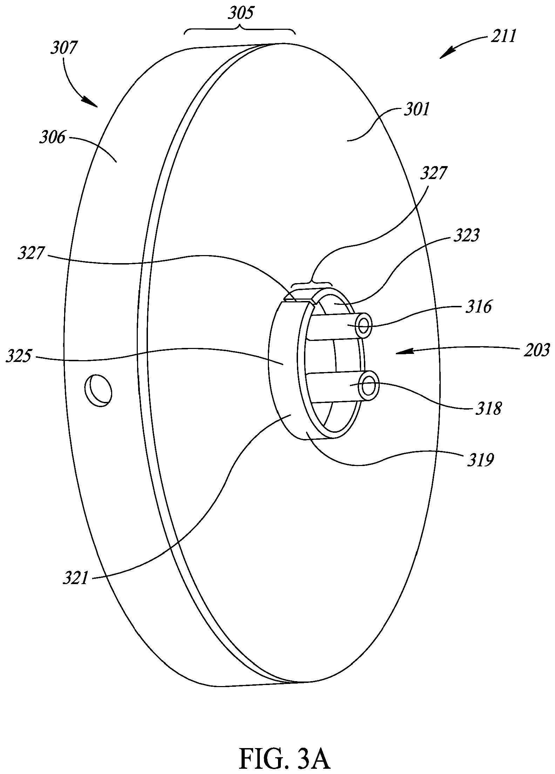

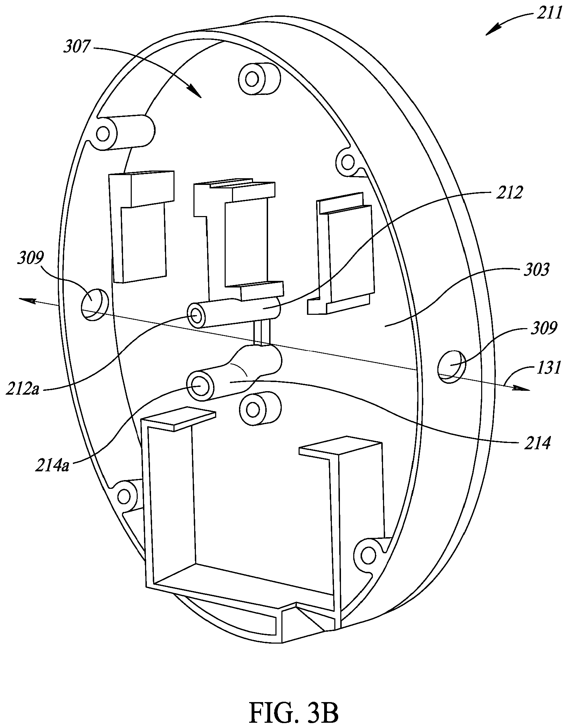

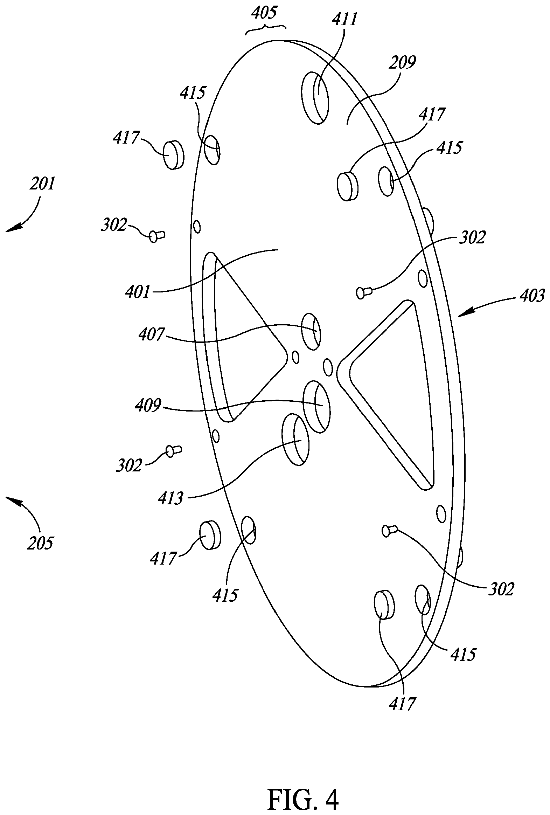

Figures 3A and 3B are isometric views of the a first side 301 and a second side 303 of the pan 211, respectively, and Figure 4 is an isometric view of the lid 209, according to at least one illustrated implementation. As previously noted, the pan 211 and the lid 209 may together form the housing 210. The first side 301 of the pan 211 faces the interior side 107 of the over-ear earpiece 101 towards the cannula 105. The second side 303 of the pan 211 faces the exterior side 109 of the over-ear earpiece 101 towards the cleaning agent reservoir 201 and the discharge collection reservoir 205. The lid 209 attaches to the second side 303 of the pan 211 via one or more coupling features, e.g., screws 302. The lid 209 (when attached to the pan 211) and the first side 301 of the pan 211 are separated by a distance 305 that forms the depth of the housing 210. A side wall 306 bridges the distance 305 between the lid 209 and the first side 301 of the pan 211. The attached lid 209, the first side 301 of the pan 211, and the side wall 306 define an interior 307 of the housing 210.

As shown in Figure 3A, the cannula coupler interface 203 may include a cleaning agent port 316, a vacuum port 318, and one or more interfaces 319. The cleaning agent port 316 may be part of the cleaning fluid fluidly communicative path 219 for the cleaning agent and may mate with a corresponding irrigation inlet port located on the cannula 105. For example, the cleaning agent port 316 may be a male connector or fastener, and the irrigation inlet port located on the cannula 105 may be a corresponding female connector or fastener. Mating the cleaning agent port 316 with the irrigation inlet port may be used to form part of a fluid path for the cleaning agent to travel from the cleaning agent reservoir 201 to the cannula 105. The vacuum port 318 may be part of the discharge fluidly communicative path 221 for the discharge and may mate with a corresponding discharge collection outlet port located on the cannula 105. For example, the vacuum port 318 may be a male connector or fastener, and the discharge collection outlet port located on the cannula 105 may be a corresponding female connector or fastener. Mating the vacuum port 318 with the discharge collection outlet port may be used to form part of a fluid path for the discharge to travel from the cannula 105 to the discharge collection reservoir 205.

The cannula coupler interface 203 may have one or more interfaces 319 that physically couple to one or more corresponding interfaces on the cannula 105. These complementary interfaces may enable the cannula 105 to be selectively detachable and physically coupled to the cannula coupler interface 203 on the over-ear earpieces 101. In some implementations, the interface 319 for the cannula coupler interface 203 may be in the form of an annular wall 321 with an interior diameter 323, an exterior diameter 325, and a height 327. The interior diameter 323 and the exterior diameter 325 may form concentric shapes (*e.g*., circles, ovals, squares, *etc*.) that encompass the cleaning agent port 316 and the vacuum port 318. The annular wall 321 may physically engage and be coupled with a corresponding interface on the cannula 105, such as, for example, a slot or opening that is sized and shaped to be only slightly larger than the annular wall 321. As such, the frictional forces may keep the cannula 105 physically coupled to the cannula coupler interface 203. In such an implementation, one or more flexible, compressible features, such as an O-ring, may be used to form an air-tight and/or water-tight seal between the coupling interfaces on the cannula coupler interface 203 and the cannula 105. In some implementations, the interface 319 may have one or more registration features that serve to properly align the cleaning agent port 316 and the vacuum port 318 of the cannula coupler interface 203 with the corresponding irrigation inlet port and discharge collection outlet port located on the cannula 105 when the interface 319 is engaged with the corresponding interface on the cannula 105. For example, the annular wall 321 may have a slot 329 that is sized and shaped to receive a corresponding tab that extends from the corresponding interface on the cannula 105. Aligning the slot 329 and the tab may be used to ensure that the cleaning agent port 316 and the vacuum port 318 of the cannula coupler interface 203 are properly aligned with the irrigation inlet port and discharge collection outlet port, respectively, located on the cannula 105.

In some implementations, the cannula coupler interface 203 may include other or additional types of interfaces 319 to physically detachably couple with the cannula 105. For example, the cannula coupler interface 203 and the cannula 105 may include complementary threads that enable the cannula 105 to be screwed onto the cannula coupler interface 203. In some implementations, the cannula 105 may be attached to the cannula coupler interface 203 using a bayonet-style coupler. In some implementations, the cannula 105 is sized and shaped to fit tightly over and couple to the cannula coupler interface 203 using one or more latches or other coupling features.

Figure 3B is an isometric view of a second side 303 of the pan 211, which may include a cleaning agent passage 212 and a discharge passage 214. The cleaning agent passage 212 may provide a first passage through the housing 210 from the cleaning agent reservoir 201 to the cannula coupler interface 203, thereby forming part of the cleaning fluid fluidly communicative path 219. The opening 212a of the cleaning agent passage 212 directed towards the cleaning agent reservoir 201 may be size, shaped, and positioned to mate with a port on the cleaning agent reservoir 201. For example, the opening 212a may form a male connector or fastener that mates with a female connector or fastener that forms a port on the cleaning agent reservoir 201. The pan 211 may include a discharge passage 214 that provides a second passage through the housing 210 from the cannula coupler interface 203 to the discharge collection reservoir 205, thereby forming part of the discharge fluidly communicative path 221. The opening 214a of the discharge passage 214 directed towards the discharge collection reservoir 205 may be size, shaped, and positioned to mate with a port on the discharge collection reservoir 205. For example, the opening 214a may form a male connector or fastener that mates with a female connector or fastener that forms a port on the discharge collection reservoir 205. In some implementations, either or both of the cleaning agent passage 212 and the discharge passage 214 may be part of the cannula 105. In such an implementation, each cannula 105 will provides its own cleaning agent passage 212 to the cleaning agent reservoir 201 and/or its own discharge passage 214 to the discharge collection reservoir 205.

In some implementations, one or both of the cleaning agent passage 212 and the discharge passage 214 are sealed from the remaining part of the interior 307 of the housing 210. Thus, the cleaning agent and the discharge proceed through the cleaning agent passage 212 and the discharge passage 214, respectively, without leaking into the interior 307 of the housing 210. In some implementations, the interior 307 of the housing 210 may enclose and be used to secure one or more additional components, such as for example the pressure pump assembly 207a, the vacuum pump assembly 207b, the controller circuitry 213, and/or the power supplies 215.

The pan 211 may include one or more coupling apertures 309 located on the side wall 306 to enable the pan 211 and/or the housing 210 to be coupled to the annular bracket 133. In some implementations, for example, the annular bracket 133 may have complementary coupling features (e.g., tabs) that correspond to the one or more coupling apertures 309 located on the side wall 306. As such, the housing 210, and by extension the over-ear earpieces 101, may be selectively detachably coupled to the annular bracket 133 via the corresponding coupling features on the pan 211 and the annular bracket 133. The coupling features on the annular bracket 133 and the corresponding coupling apertures 309 in the housing 210 may form a set of pivot couplers 130 that, as discussed above, enable the over-ear earpieces 101 to pivot relative to the axis 131 that runs between the coupling features.

Figure 4 shows the lid 209, which has a first side 401 and an opposing second side 403 separated by a distance 405, according to at least one illustrated implementation. The first side faces the exterior side 109 of the over-ear earpiece 101 towards the cleaning agent reservoir 201 and the discharge collection reservoir 205. The second side 403 faces the interior side 107 of the over-ear earpiece 101 towards the pan 211 and the cannula coupler interface 203. The lid 209 may include a cleaning agent aperture 407 that enables the cleaning agent to pass from the cleaning agent reservoir 201 to the cannula 105. The lid 209 may include a discharge aperture 409 that enables the discharge to pass from the cannula 105 to the discharge collection reservoir 205. The lid 209 may include a pump aperture 411 that enables the output of the pump assembly 207a, located on the second side 403 of the lid 209, to be physically coupled to the cleaning agent reservoir 201, located on the first side 401 of the lid 209. The pump assembly 207a may include a pressure pump and/or manifold operable to generate a positive pressure (*e.g*., greater than one atmosphere or greater than ambient pressure) within the cleaning agent reservoir 201. As shown in Figure 4, the pump aperture 411 may be located proximate the edge of the lid 209. The lid 209 may include a vacuum aperture 413 that enables the vacuum assembly 207b, located on the second side 403 of the lid 209, to be physically mated with the discharge collection reservoir 205, located on the first side 401 of the lid 209. The vacuum assembly 207b may include a vacuum pump and/or manifold operable to generate a negative pressure (*e.g*., less than one atmosphere or less than ambient pressure) within the discharge collection reservoir 205 to draw discharge from the cannula 105 out of the auditory canal and into the discharge collection reservoir 205.

The lid 209 includes one or more coupling features 415. The coupling features 415 are used to secure one o both of the cleaning agent reservoir 201 and the discharge collection reservoir 205 to the lid 209. In some implementations, the coupling features 415 may be magnets and/or ferromagnetic pieces, such as magnets 417, that have corresponding magnets of opposite polarity in either the cleaning agent reservoir 201 or the discharge collection reservoir 205. The use of magnets 417 as the coupling feature 415 may advantageously enable a user to quickly and easily remove the cleaning agent reservoir 201 for filling and/or the discharge collection reservoir 205 for emptying while also ensuring that both the cleaning agent reservoir 201 and the discharge collection reservoir 205 can be securely coupled to the over-ear earpiece 101.

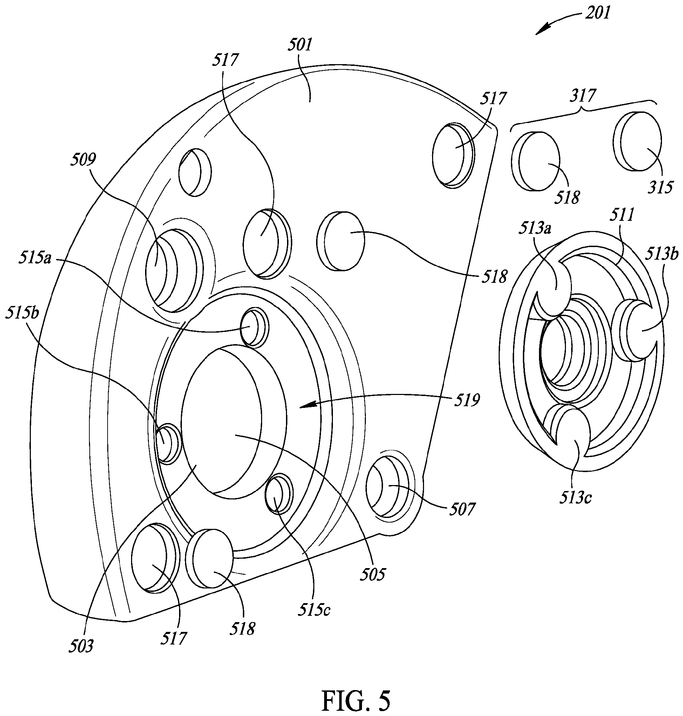

Figure 5 is an isometric view of the cleaning agent reservoir 201, according to at least one illustrated implementation. The cleaning agent reservoir 201 may include a shell 501 having a plurality of sides that define an enclosed space 503 in which the cleaning agent is held. In some implementations, the enclosed space 503 may have a volume that holds sufficient cleaning agent for performing one or more irrigation procedures. In some implementations, for example, the enclosed space 503 may hold up to 100 mL of cleaning agent. The shell 501 may include multiple apertures, including a loading aperture 505, a cleaning agent port 507, and a pressure aperture 509.

The loading aperture 505 may be used to load the cleaning agent into the enclosed space 503. In some implementations, the loading aperture 505 may be mated with and sealed by a cleaning agent cover 511. In some implementations, the cover 511 may include one or more magnets 513a, 513b, and 513c that magnetically couple to corresponding magnets 515a, 515b, and 515c of the opposite polarity, thus providing multiple pairs of ferromagnetic couplers spaced proximate the edge of the loading aperture 505. Alternatively or in addition, in some implementations, the loading aperture 505 and the cleaning agent cover 511 may have complementary threads that enable the cleaning agent cover 511 to be screwed into the loading aperture 505. In some implementations, the cover 511 may include a protrusion that is sized and shaped to fit tightly with a corresponding slot or opening proximate the edge of the loading aperture 505. In some implementations, such a protrusion for the cover 511 may be sized and shaped to fit tightly inside the loading aperture 505 itself. A flexible, water-tight material (*e.g*., an O-ring) may be placed to go around the portion of the cover 511 that is adjacent to the edge of the loading aperture 505 for a water-tight and air-tight seal. As such, in some implementations, the cleaning agent reservoir 201 may be formed by the shell 501 and the cleaning agent cover 511.

In some implementations, the cleaning agent port 507 is sized and shaped to mate with the opening 212a of the cleaning agent passage 212 that extends through the housing 210, thus providing an exit for the cleaning agent from the enclosed space 503. In some implementations, the pressure aperture 509 is mated to an output of the pump assembly 207a. In such implementations, the pump assembly 207a may be used to increase the pressure inside the enclosed space 503, thus forcing the cleaning agent through the cleaning agent port 507 at increased pressure. In some implementations, the pump assembly 207a may be a water or liquid pump that draws cleaning agent directly from the cleaning agent port 507 and dispenses it through the cleaning agent passage 212 at a specified positive pressure. In such an implementation, the cleaning agent reservoir 201 may not include the pressure aperture 509. The pressure for the pump assembly 207a may be set so as to not cause discomfort to the user when the cleaning agent exits the cannula 105. In some implementations, for example, the pressure for the pump assembly 207a may be less than about 100 kPa.

The shell 501 may include one or more coupling features 517 that couple with the corresponding coupling features 311 located on the lid 209 and or the housing 210, thereby securing the shell 501 to the housing 210 as discussed above. In some implementations, the coupling features 517 may include magnets 518 that magnetically couple with the corresponding magnets 315 of opposite polarity located on the housing 210. Each magnetic pair may thereby form a pair of ferromagnetic couplers 317. The ferromagnetic couplers 317 may be used to selectively, detachably, magnetically couple the shell 501 and the cleaning agent reservoir 201 to the housing 210.

In some implementations, the loading aperture 505 may be inside a depression or well 519 placed into the shell 501. Accordingly, when the cleaning agent cover 511 is secured in the shell 501, the cleaning agent cover 511 may be at or below a plane formed by the side of the shell 501 to which the cleaning agent cover 511 attaches. As such, the cover 511 may not impede or interfere with the magnetic coupling of the ferromagnetic couplers 317.

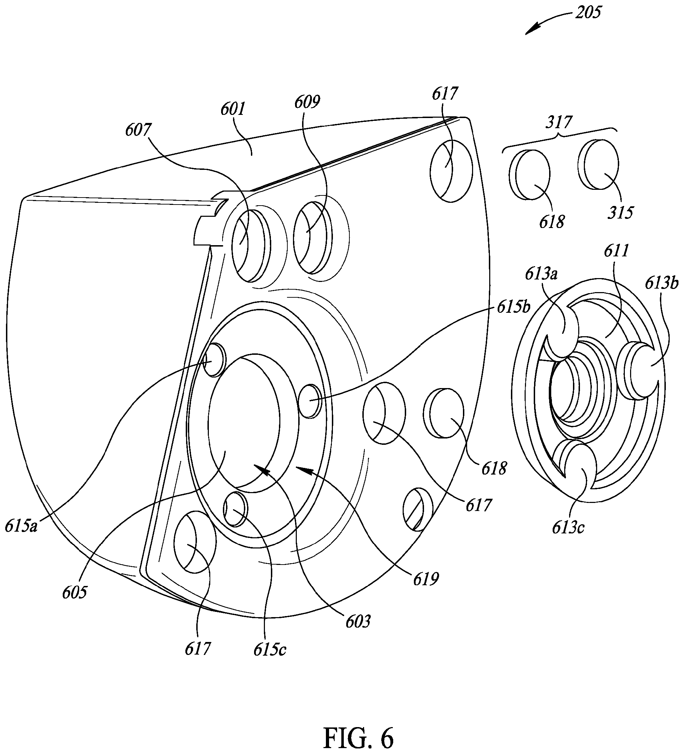

Figure 6 is an isometric view of the discharge collection reservoir 205, according to at least one illustrated implementation. As shown in Figure 6, the discharge collection reservoir 205 may include a shell 601 having a plurality of sides that define an enclosed space 603 in which the discharge from the irrigation is held. In some implementations, the enclosed space 603 may have a volume that is slightly larger than the volume of cleaning agent held by the cleaning agent reservoir 201. The shell 601 may include multiple apertures, including a discharge removal aperture 605, a discharge port 607, and a vacuum aperture 609.

The discharge removal aperture 605 may be used to remove the discharge from the enclosed space 603. In some implementations, the discharge removal aperture 605 may be mated with and sealed by a discharge cover 611. In some implementations, for example, the discharge cover 611 may include one or more magnets 613a, 613b, and 613c that magnetically couple to corresponding magnets 615a, 615b, and 615c of the opposite polarity, thus providing multiple pairs of ferromagnetic couplers spaced proximate the edge of the discharge removal aperture 605. Alternatively or in addition, the discharge removal aperture 605 and the discharge cover 611 may have complementary threads that enable the discharge cover 611 to be screwed into the discharge removal aperture 605. In some implementations, the discharge cover 611 may include a protrusion that is sized and shaped to fit tightly with a corresponding slot or opening proximate to the edge of the discharge removal aperture 605. In some implementations, such a protrusion for the discharge cover 611 may be sized and shaped to fit tightly inside the discharge removal aperture 605 itself. A flexible, water-tight material (e.g., an elastomeric O-ring) may go around the portion of the discharge cover 611 that is adjacent to the edge of the discharge removal aperture 605 to form a water-tight and air-tight seal. As such, in some implementations, the discharge collection reservoir 205 may be formed by the shell 601 and the discharge cover 611.

In some implementations, the discharge port 607 is sized and shaped to mate with the opening 214a of the discharge passage 214 that extends through the housing 210, thus providing an exit for the discharge to travel to the enclosed space 603. In some implementations, the vacuum aperture 609 is mated to an output of the vacuum assembly 207b. In such implementations, the vacuum assembly 207b may be used to form an area of low pressure inside the enclosed space 603, thus drawing discharge from the discharge port 607 into the enclosed space 603. The vacuum pressure for the vacuum assembly 207b may be set for the comfort and/or safety of the user. In some implementations, for example, the vacuum pressure for the vacuum assembly 207b may be less than about 100 kPa.

The shell 601 may include one or more coupling features 617 that couple with corresponding coupling features 311 located on the housing 210, thereby securing the shell 601 to the housing 210 as discussed above. In some implementations, the coupling features 617 may include magnets 618 that magnetically couple with corresponding magnets 315 of opposite polarity located on the housing 210, thereby forming a pair of ferromagnetic couplers 317. The ferromagnetic couplers 317 may be used to detachably, magnetically couple the shell 601 and the discharge collection reservoir 205 to the housing 210. Further, in some implementations, the shell 501 for the cleaning agent and the shell 601 for the discharge may be independently coupled to the housing 210. In such implementations, each of the cleaning agent shell 501 and the discharge shell 601 may be selectively and independently detached from the housing 210 with respect to the other.

In some implementations, the removal aperture 605 may be inside a depression or well 619 placed into the shell 601. Accordingly, when the discharge cover 611 is secured in the shell 601, it may be at or below a plane formed by the side of the shell 601 to which the discharge cover 611 attaches. As such, the discharge cover 611 may not impede or interfere with the magnetic coupling of the ferromagnetic couplers 317.

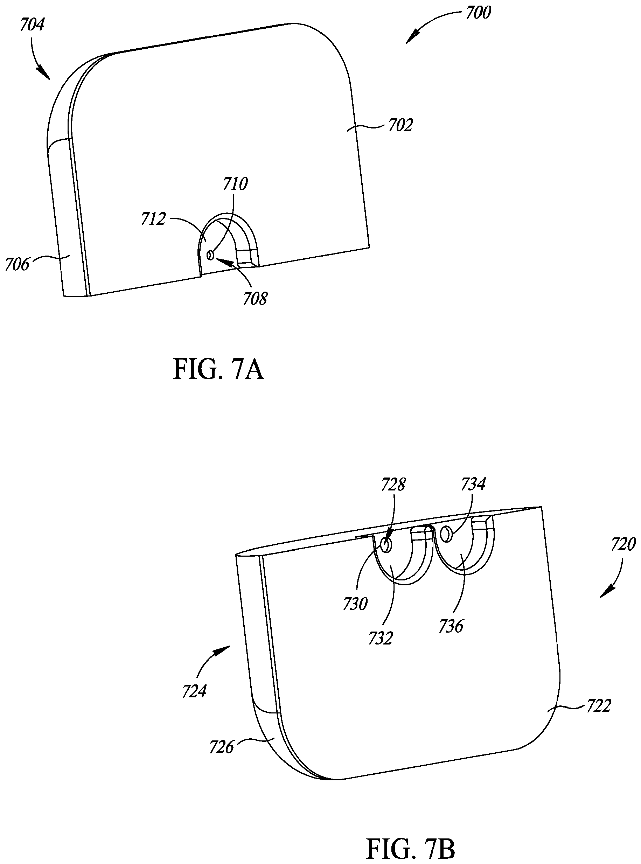

Figure 7A is an isometric view of a disposable cleaning agent reservoir 700 of an over-ear earpiece 101, according to at least one illustrated implementation. The disposable cleaning agent reservoir 700 may include a front face 702 and a back face 704. In some implementations, the front face 702 and the back face 704 may be substantially parallel to each other. In such implementations, the disposable cleaning agent reservoir 700 may include the one or more side walls 706 that bridge the distance between the front face 702 and the back face 704. In some implementations, the front face 702 and the back face 704 may meet at an edge such that the back face 704 curves outward from the front face 702 and/or the front face 702 curves outward from the back face 702. In such an implementation, a cross-sectional area of the disposable cleaning agent reservoir 700 may include an arc along one or both of the sides associated with the back face 704 and the front face 702.

The front face 702, the back face 704, and the side walls 706 (when present) may define a hollow space 708 for the disposable cleaning agent reservoir 700, with each of the front face 702, the back face 704, and the side walls 706 delineating a boundary for the hollow space. In some implementations, the hollow space 708 may be accessible via a disposable cleaning agent reservoir port 710 located within a depression 712 located on the front face 702 of the disposable cleaning agent reservoir 700. The disposable cleaning agent reservoir port 710 may be sized and dimensioned to selectively, detachably, physically mate with the cleaning fluid fluidly communicative path 219 at an end opposite the cannula 105, e.g., at the opening 212a of the cleaning agent passage 212. When the disposable cleaning agent reservoir port 710 is mated with the cleaning fluid fluidly communicative path 219, the disposable cleaning agent reservoir 700 may provide cleaning agent for one or more irrigation procedures. In some implementations, the disposable cleaning agent reservoir port 710 may be fluidly coupled to a pump assembly 207a that is a water pump that draws cleaning agent out of the disposable cleaning agent reservoir 700 towards the cannula 105. In some implementations, the disposable cleaning agent reservoir port 710 may be sealed with a flexible membrane during a manufacturing and/or assembly process after cleaning agent has been placed into the hollow space 708 of the disposable cleaning agent reservoir 700. In some implementations, the cleaning fluid fluidly communicative path 219 (e.g., the tip of the cleaning agent passage 212) may puncture the membrane thereby accessing the cleaning fluid when the disposable cleaning agent reservoir 700 is mounted onto an over-ear earpiece 101.

The disposable cleaning agent reservoir 700 may be made of any suitable plastic materials, such as, for example, plastic material that is approved for use in medical procedures. In some implementations, the disposable cleaning agent reservoir 700 may hold sufficient cleaning agent to perform one (1) irrigation cycle of an irrigation routine. Such an irrigation cycle may be sufficient for cleaning one ear of a human user, for example. Accordingly, such a disposable cleaning agent reservoir 700 may be changed out after every use, thereby reducing the number of components of the irrigation device 100 exposed to multiple users. In some implementations, the disposable cleaning agent reservoir 700 may be mounted directly onto the over-ear earpiece 101 using one or more fasteners or physical couplers. In some implementations, as discussed below, the disposable cleaning agent reservoir 700 may be loaded into a shell and mounted on the over-ear earpiece 101 when the shell is attached to the over-ear earpiece.

Figure 7B is an isometric view of a disposable discharge collection reservoir of an over-ear earpiece 720, according to at least one illustrated implementation. The disposable discharge collection reservoir 720 may include a front face 722 and a back face 724. In some implementations, the front face 722 and the back face 724 may be substantially parallel to each other. In such implementations, the disposable discharge collection reservoir 720 may include the one or more side walls 726 that bridge the distance between the front face 722 and the back face 724. In some implementations, the front face 722 and the back face 724 may meet at an edge such that the back face 724 curves outward from the front face 722 and/or the front face 722 curves outward from the back face 722. In such an implementation, a cross-sectional area of the disposable discharge collection reservoir 720 may include an arc along one or both of the sides associated with the back face 724 and the front face 722.

The front face 722, the back face 724, and the side walls 726 (when present) may define a hollow space 728 for the disposable discharge collection reservoir 720, with each of the front face 722, the back face 724, and the side walls 726 delineating a boundary for the hollow space 728. In some implementations, the hollow space 728 may be accessible via a disposable discharge collection reservoir port 730 located within a first depression 732 located on the front face 722 of the disposable discharge collection reservoir 720. The disposable discharge collection reservoir port 730 may be sized and dimensioned to selectively, detachably, physically mate with the discharge fluidly communicative path 221 at an end opposite the cannula 105, e.g., at the opening 214a of the discharge passage 214. When the disposable discharge collection reservoir port 730 is mated with the discharge fluidly communicative path 221, the disposable discharge collection reservoir 720 may be used to collect discharge during one or more irrigation procedures. In some implementations, the disposable discharge collection reservoir port 730 may be sealed with a flexible membrane during a manufacturing and/or assembly process. In some implementations, the discharge fluidly communicative path 221 (*e.g*., the tip of the discharge passage 214) may puncture the membrane thereby providing access to the hollow space 728 of the disposable discharge collection reservoir 720 when the disposable discharge collection reservoir 720 is mounted onto an over-ear earpiece 101.

The front face 722 of the disposable discharge collection reservoir 720 may include a vacuum assembly port 734 that may provide access to the hollow space 728 of the disposable discharge collection reservoir 720. The vacuum assembly port 734 may be located within a second depression 736 on the front face 722 of the disposable discharge collection reservoir 720. The vacuum assembly port 734 may be sized and dimensioned to physically, fluidly couple with the vacuum assembly 207b when the disposable discharge collection reservoir 720 is mounted onto an over-ear earpiece 101. The vacuum assembly or vacuum pump 207b may be used to create a vacuum or area of low pressure (*i.e*., lower than ambient environmental pressure, e.g., lower than 1 atmosphere) within the disposable discharge collection reservoir 720 to facilitate the removal of the discharge from the auditory canal during and/or after an irrigation procedure.

The disposable discharge collection reservoir 720 may be made of any suitable plastic materials, such as, for example, plastic material that is approved for use in medical procedures. In some implementations, the hollow space 728 of the disposable discharge collection reservoir 720 may hold sufficient volume to contain discharge from at least one (1) irrigation cycle of an irrigation routine. Such an irrigation cycle may be sufficient for cleaning one ear of a human user, for example. Accordingly, such a disposable discharge collection reservoir 720 may be changed out after every use, thereby reducing the number of components of the irrigation device 100 exposed to multiple users. In some implementations, the disposable discharge collection reservoir 720 may be mounted directly onto the over-ear earpiece 101 using one or more fasteners or physical couplers. In some implementations, as discussed below, the disposable discharge collection reservoir 720 may be loaded into a shell and mounted on the over-ear earpiece 101 when the shell is attached to the over-ear earpiece.

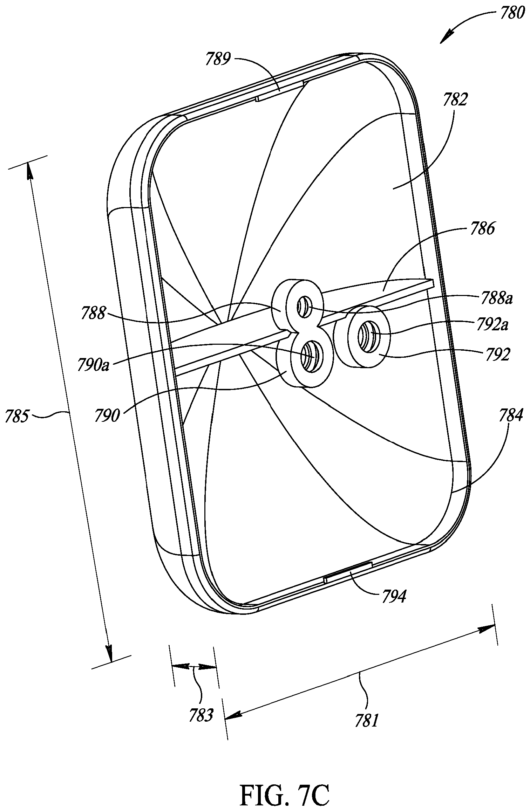

Figure 7C is an isometric view of a reservoir shell 780 broken into two reservoir cavities, a first cavity 782 which is sized and dimensioned to hold a disposable cleaning agent reservoir 700, and a second cavity 784 is sized and dimensioned to hold a disposable discharge collection reservoir 720, according to at least one illustrated implementation. The reservoir shell 780 may have a length 781, a width 783, and a height 785, and may be sized and dimensioned to fit over an exposed portion of an over-ear earpiece 101. The reservoir shell 780 includes an interior wall 786 that runs across the length 781 of the reservoir shell 780 and separates the first cavity 782 from the second cavity 784. A first collar 788 may be attached to the interior wall 786 and extend perpendicularly to the interior wall 786 towards the first cavity 782. The first collar 788 may have an annular shape with an interior opening 788a. In some implementations, the interior opening 788a may align with the disposable cleaning agent reservoir port 710 when the disposable cleaning agent reservoir 700 is loaded into the first cavity 782. Such alignment may facilitate the mating of the disposable cleaning agent reservoir port 710 with the cleaning fluid fluidly communicative path 219.

In some implementations, the disposable cleaning agent reservoir 700 may be loaded into the first cavity 782 by sliding the end of the disposable cleaning agent reservoir 700 that has the disposable cleaning agent reservoir port 710 into the first cavity 782 towards the interior wall 786 and behind the first collar 788. In some implementations, the reservoir shell 780 may optionally have a latch 789 or other similar securing feature located along the edge of the first cavity 782 opposite the interior wall 786. The corresponding end of the disposable cleaning agent reservoir 700 may be pressed into first cavity 782 behind the latch 789 to thereby secure the disposable cleaning agent reservoir 700 into the first cavity 782.

A second collar 790 and a third collar 792 may be attached to the interior wall 786 and extend perpendicularly to the interior wall 786 towards the second cavity 784. The second collar 790 may have an annular shape with an interior opening 790a. In some implementations, the interior opening 790a may align with the disposable discharge collection reservoir port 730 when the disposable discharge collection reservoir 720 is loaded into the second cavity 784. Such alignment may facilitate the mating of the disposable discharge collection reservoir port 730 with the discharge fluidly communicative path 221. The third collar 792 may have an annular shape with an interior opening 792a. In some implementations, the interior opening 792a may align with an output from the vacuum assembly 207b when the disposable discharge collection reservoir 720 is loaded into the second cavity 784. Such alignment may facilitate the mating of the vacuum assembly port 772 with the vacuum assembly 207b.

In some implementations, the disposable discharge collection reservoir 720 may be loaded into the second cavity 784 by sliding the end of the disposable discharge collection reservoir 720 that has the disposable discharge collection reservoir port 730 into the second cavity 784 towards the interior wall 786 and behind the second collar 790 and/or third collar 792. In some implementations, the reservoir shell 780 may optionally have a latch 794 or other similar securing feature located along the edge of the second cavity 784 opposite the interior wall 786. The corresponding end of the disposable discharge collection reservoir 720 may be pressed into second cavity 784 behind the latch 794 to thereby secure the disposable discharge collection reservoir 720 into the second cavity 784.

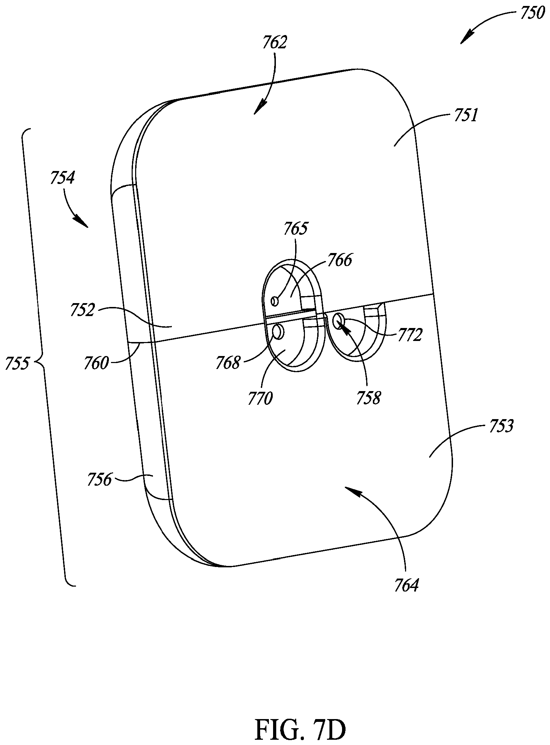

Figure 7D is an isometric view of a disposable container 750 that includes separate sections for a cleaning agent section 751 and a discharge collection section 753, according to at least one illustrated implementation. The disposable container 750 may include a front face 752 and a back face 754, and potentially one or more side walls 756, that form a unitary body 755. In some implementations, the front face 752 and the back face 754 may be substantially parallel to each other. In such implementations, disposable container 750 may include one or more side walls 756 that bridge the distance between the front face 752 and the back face 754. In some implementations, the front face 752 and the back face 754 may meet at an edge such that the back face 754 curves outward from the front face 752 and/or the front face 752 curves outward from the back face 752. In such an implementation, a cross-sectional area of the disposable container 750 may include an arc along one or both of the sides associated with the back face 754 and the front face 752.

The front face 752, the back face 754, and the side walls 756 (when present) may define a hollow space 758 for the disposable container 750, with each of the front face 752, the back space 754, and the side walls 756 delineating a boundary for the hollow space 758. In some implementations, a partition 760 may divide the hollow space 758 into multiple sections. In such implementations, the different sections may be fluidly separated from each other such that the fluid contents of one section would not be able to travel directly within the disposable container 750 to another section. For example, in some implementations, the hollow space 758 may be divided into a cleaning agent reservoir section 762 and a discharge collection reservoir section 764, with such sections fluidly separated from the other within the disposable container 750.

In some implementations, the cleaning agent reservoir section 762 of the hollow space 758 may be accessible via a cleaning agent port 765 located within a first depression 766 on the front face 702 of the disposable container 750. The cleaning agent port 765 may be sized and dimensioned to selectively, detachably, physically mate with the cleaning fluid fluidly communicative path 219 at an end opposite the cannula 105, e.g., at the opening 212a of the cleaning agent passage 212. When the disposable container 750 is mated with the cleaning fluid fluidly communicative path 219, the cleaning agent reservoir section 762 may provide cleaning agent for one or more irrigation procedures. In some implementations, the cleaning agent port 765 may be sealed with a flexible membrane during a manufacturing and/or assembly process after cleaning agent has been placed into the cleaning agent reservoir section 762 of the disposable container 750. In some implementations, the cleaning fluid fluidly communicative path 219 (e.g., the tip of the cleaning agent passage 212) may puncture the membrane thereby accessing the cleaning fluid when the disposable container 750 is mounted onto an over-ear earpiece 101.

In some implementations, the discharge collection reservoir section 764 may be accessible via a discharge collection reservoir port 768 located within a second depression 770 on the front face 752 of the disposable container 750. The discharge collection reservoir port 768 may be sized and dimensioned to selectively, detachably, physically mate with the discharge fluidly communicative path 221 at an end opposite the cannula 105, e.g., at the opening 214a of the discharge passage 214. When the discharge collection reservoir port 768 is mated with the discharge fluidly communicative path 221, the discharge collection reservoir section 764 may be used to collect discharge during one or more irrigation procedures. In some implementations, the discharge collection reservoir section 764 may be sealed with a flexible membrane during a manufacturing and/or assembly process. In some implementations, the discharge fluidly communicative path 221 (*e.g*., the tip of the discharge passage 214) may puncture the membrane thereby providing access to the discharge collection reservoir section 764 when the disposable container 750 is mounted onto an over-ear earpiece 101.

The front face 752 of the disposable container 750 may include a vacuum assembly port 772 that may provide access to the discharge collection reservoir section 764 of the disposable container 750. The vacuum assembly port 772 may be located within a third depression 774 on the front face 752 of the disposable container 750. The vacuum assembly port 772 may be sized and dimensioned to physically, fluidly couple with the vacuum assembly 207b when the disposable container 750 is mounted onto an over-ear earpiece 101. The vacuum assembly or vacuum pump 207b may be used to create a vacuum or area of low pressure (*i.e.*, lower than ambient environmental pressure, e.g., lower than 1 atmosphere) within the discharge collection reservoir section 764 to facilitate the removal of the discharge from the auditory canal during and/or after an irrigation procedure.

The disposable container 750 may be made of any suitable plastic materials, such as, for example, plastic material that is approved for use in medical procedures. In some implementations, the cleaning agent reservoir section 762 of the disposable container 750 may hold sufficient volume to contain discharge from at least one (1) irrigation cycle of an irrigation routine. In some implementations, the discharge collection reservoir section 764 may hold sufficient volume to contain discharge from at least one (1) irrigation cycle of an irrigation routine. Such an irrigation cycle may be sufficient for cleaning one ear of a human user, for example. Accordingly, such a disposable container 750 may be changed out after every use, thereby reducing the number of components of the irrigation device 100 exposed to multiple users. In some implementations, the disposable container 750 may be mounted directly onto the over-ear earpiece 101 using one or more fasteners or physical couplers. In some implementations, the disposable container 750 may be loaded into a shell (*e.g.*, reservoir shell 780 without interior wall 786) and mounted on the over-ear earpiece 101 when the shell is attached to the over-ear earpiece.

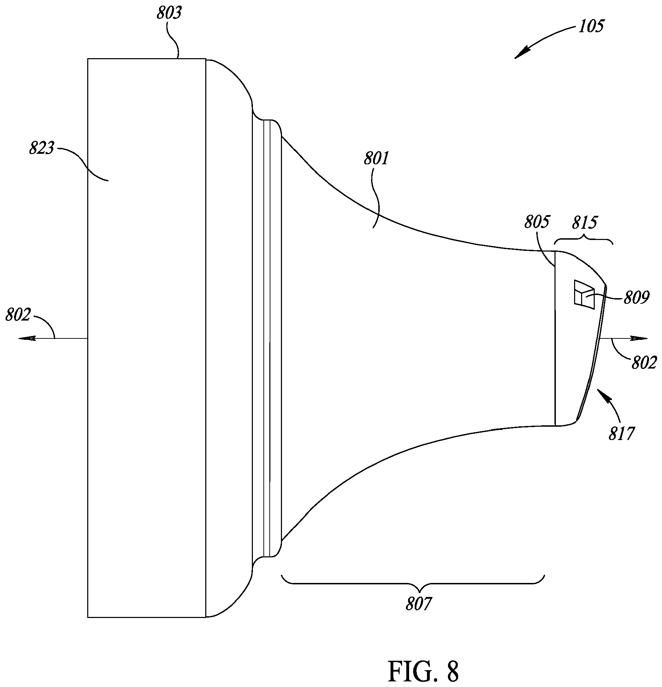

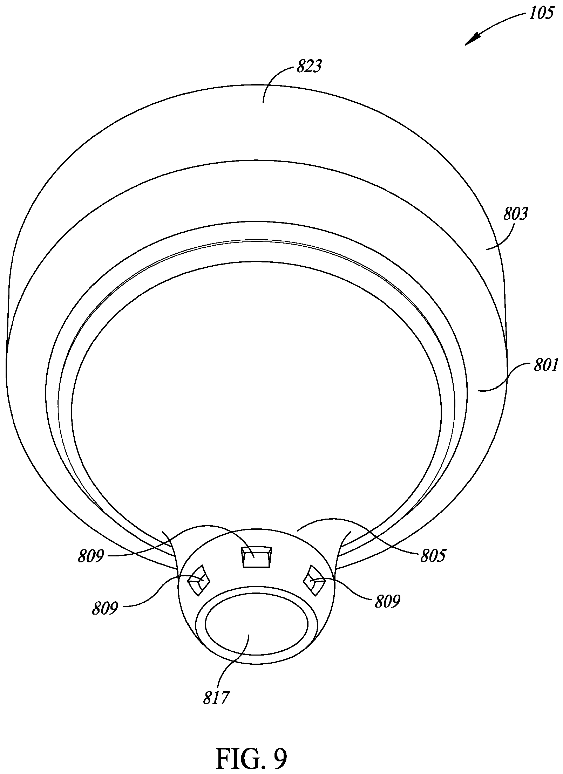

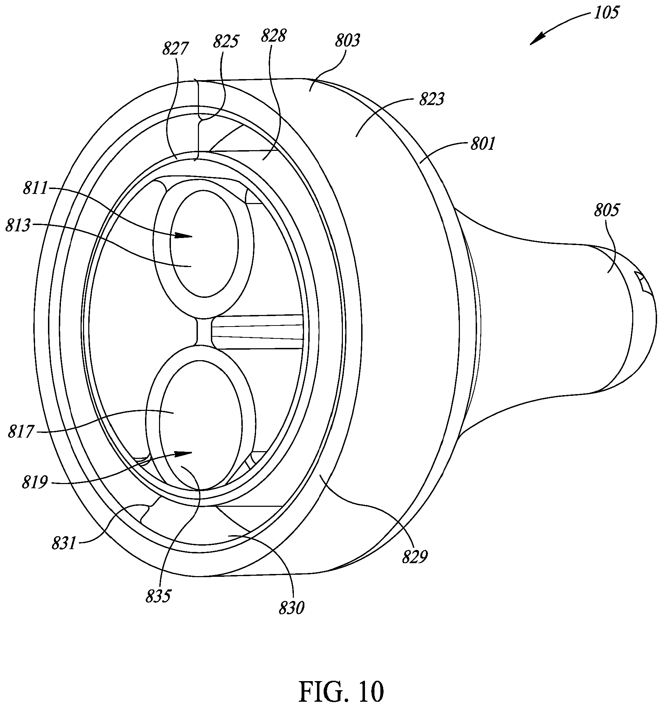

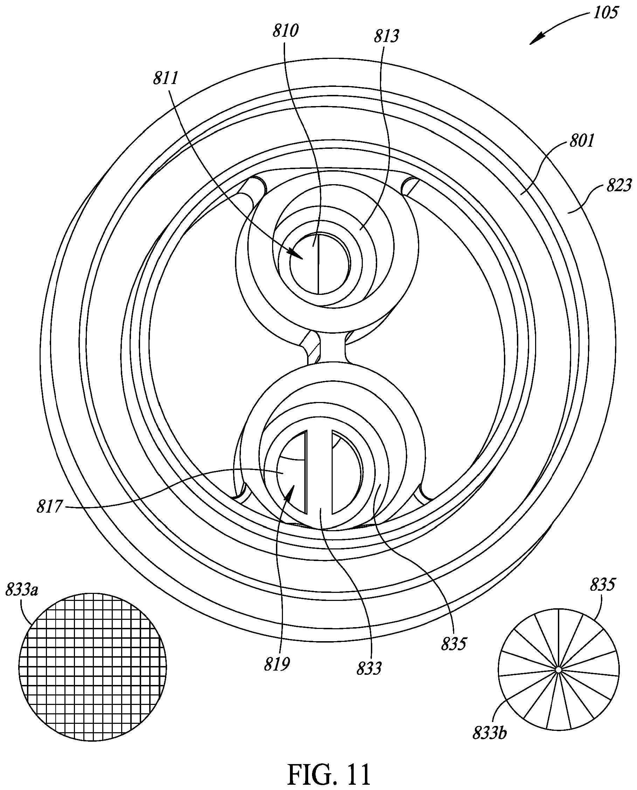

Figures 8, 9, 10, and 11 show a cannula 105 from various perspectives, according to one implementation. The cannula 105 includes a body 801 having a proximal end 803 and a distal end 805 separated by and opposing each other across a length 807. The body 801 may taper from the proximal end 803 towards the distal end 805, such that a cross-sectional area of the proximal end 803 is greater than a cross-sectional area of the distal end 805, which is inserted into the user's auditory canal. In such an implementation, the tapering of the cannula 105 may protect a user's ear drum by having a relatively larger portion of the body 801 impact a side wall of the user's auditory canal before the distal end 805 of the cannula 105 impacts the user's ear drum. In some implementations, the body 801 of the cannula 105 may have a partially conical shape, such as a frustro-conical shape in which the tapered end of the cone has been truncated. In some implementations, the tapering of the body 801 of the cannula 105 may be linear. In some implementations, the tapering of the body 801 of the cannula 105 may be non-linear, with a curve and/or steps in the profile as traversed from base to tip. In some implementations, the tapering may be linear over some portion of the body 801 of the cannula 105 and non-linear over other portions of the body 801 of the cannula 105. In some implementations, the body 801 of the cannula 105 may be formed of a unitary single-piece of plastic. In some implementations, alternatively, the body 801 of the cannula 105 may be formed of a plurality of pieces that have been joined together. In some implementations, the body 801 may have a form that is a body of rotation about a central axis 802. In some implementations, the body 801 of the cannula 105 may not be in the form of a body of rotation.

One or more irrigation outlet apertures 809 may be located proximate the distal end 805. In some implementations, one or more of the irrigation outlet apertures 809 may be set back a short distance (e.g., up to 5 mm) from the edge at the distal end 805 of the cannula 105. The irrigation outlet apertures 809 may be used to direct an outward flow of cleaning agent that is exiting from the cannula 105. In some implementations, multiple irrigation outlet apertures 809 may be radially spaced around the body 801 of the cannula 105 proximate the distal end 805. The irrigation outlet aperture 809 may be located at the end of an irrigation passage 810 that provides an irrigation flow path 811 between an irrigation inlet port 813 located at the proximal end 803 of the cannula 105 and the irrigation outlet aperture 809 located relatively towards the distal end 805 with respect to the irrigation inlet port 813. The irrigation inlet port 813 may be substantially cylindrical in shape, or may have other shapes, for instance oval or elliptical.

A discharge collection inlet port 817 may be located at, or at least proximate, the distal end 805 of the cannula 105. The discharge collection inlet port 817 may be used to collect the discharge from the user's auditory canal during an irrigation procedure. In some implementations, a suction force may be created at the discharge collection inlet port 817 via a vacuum or area of relatively low or negative air pressure created by the vacuum assembly 207b within the discharge collection reservoir 205. The suction force may assist in collecting the discharge. The discharge collection inlet port 817 may be located at the end of a discharge flow path 819 that provides a flow path for the discharge between the discharge collection inlet port 817 located relatively towards the distal end 805 of the cannula 105 and the discharge collection outlet port 821 located at the proximal end 803 of the cannula 105. The discharge collection outlet port 821 may be substantially cylindrical in shape. In some implementations, the discharge collection outlet port 821 may take other shapes (*e.g*., oval, elliptical, *etc*.).

In some implementations, the irrigation inlet port 813 is radially offset from the discharge collection outlet port 821. In some implementations, the irrigation outlet apertures 809 may be radially offset outwardly from the central axis 802; such placement may result in the irrigation outlet apertures 809 forming an arc. In some implementations, the discharge collection inlet port 817 may be disposed about the central axis 802.

A trap 833 may be located along the discharge flow path 819. In some implementations, for example, the trap 833 may be located proximate the proximal end 803 of the cannula 105. In some implementations, the trap 833 may be sized and dimensioned to trap physical debris of at least one defined dimension while passing at least one of a quantity of a liquid and air. For example, the trap 833 may be sized and dimensioned to trap ear wax being carried in the discharge. In some implementations, the trap 833 may be, for example, a filter 833a, for instance a mesh filter, woven filter, or non-woven filter, that extends across discharge flow path 819 and is oriented to be perpendicular to the direction of flow of the discharge in the discharge flow path 819. In some implementations, the trap 833 may include a plurality of fingers or projections 833b that extend radially inward from an inside perimeter 835 of the discharge collection passage 820. While a plurality of fingers or projections 833b are illustrated, the trap can comprise a single bar or rod or elongated member that extends all the way or at least partially across the discharge collection passage 820.

In some implementations, the distal end 805 of the cannula 105 may include a beveled portion 815 that overhangs at least a portion of the discharge collection inlet port 817. In such implementations, the beveled portion 815 may position the discharge collection inlet port 817 such that it is directed at an angle with respect to the central axis 802, lying in a plane that is neither perpendicular nor parallel with the central axis 802. In some implementations, the beveled portion 815 may be shaped to orient the discharge collection inlet port 817 at a downward angle with respect to the horizontal axis 104 when a user in an upright position wears the irrigation device 100. Such an orientation may improve the effectiveness and efficiency of the suction introduced at the discharge collection inlet port 817 to collect the discharge of the irrigation procedure. In some implementations, the beveled portion 815 may include one or a plurality of the irrigation outlet apertures 809.

The proximal end 803 may include a flanged portion 823. In some implementations, the flanged portion 823 may include one or more interfaces to securely engage the cannula 105 to complementary interfaces on the cannula coupler interface 203, thereby securing the cannula 105 to the over-ear earpiece 101. In some implementations, the flanged portion 823 may include an interface 825 formed by an interior wall 827 that has an exterior diameter 828, an exterior wall 829 that has an interior diameter 830, and an open space 831 between the exterior diameter 828 and the interior diameter 830. In such an implementation, the cannula coupler interface 203 may include a corresponding, complementary interface 319, such as the annular wall 321, that is designed and shaped to fit tightly into the open space 831 and make contact with the exterior wall 829 and the interior wall 827. The cannula coupler interface 203 may securely hold the cannula 105 in place via frictional forces that will oppose movement of the cannula 105 when the annular wall 321 on the cannula coupler interface 203 is engaged between the interior wall 827 and the exterior wall 829. Such frictional forces, though, may not be so great as to prevent a user from removing the cannula 105 from the cannula coupler interface 203 when desired. Accordingly, such an interface 825 removably, securely engages the cannula 105 with the complementary interface 319 on the cannula coupler interface 203.

The flanged portion 823 may include other types of interfaces (*e.g*., a threaded screw, a bayonet connector (*e.g*., lugs and complementary recesses) that enable the cannula 105 to be removably, securely engaged with the cannula coupler interface 203. The flanged portion 823 may include one or more flexible, conformable materials (*e.g*., rubber O-rings) that provide for a water tight and/or airtight seal between the cannula 105 and the cannula coupler interface 203.

The interface 825 may align the irrigation inlet port 813 located on the proximal end 803 of the cannula 105 with the cleaning agent port 316 located on the cannula coupler interface 203. The interface 825 may align the discharge collection outlet port 821 located on the proximal end 803 of the cannula 105 with the vacuum port 318 located on the cannula coupler interface 203. In such an implementation, when the cannula 105 is secured to the cannula coupler interface 203, the interface may provide that the irrigation inlet port 813 securely mates with the cleaning agent port 316, and that the discharge collection outlet port 821 securely mates with the vacuum port 318. One or more flexible, conformable seals may further be used to provide an air-tight and/or water-tight seal between irrigation inlet port 813 and the cleaning agent port 316, and/or the discharge collection outlet port 821 and the vacuum port 318.

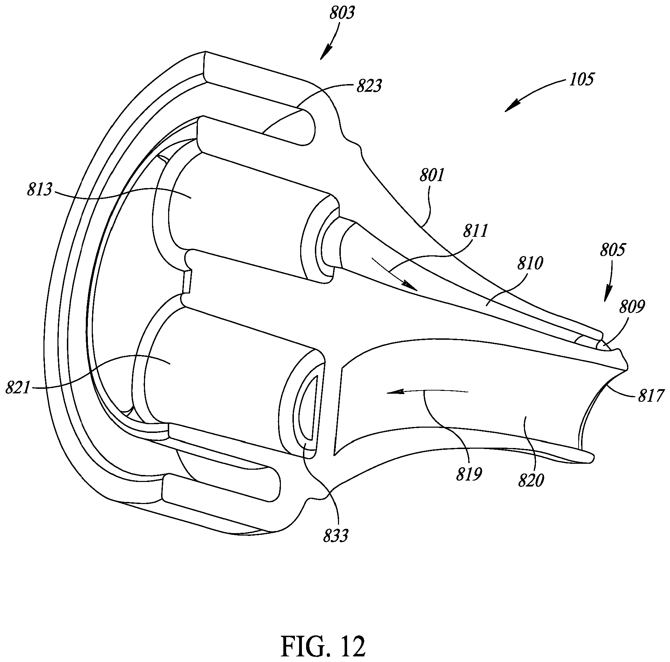

Figure 12 shows cannula 105 and better illustrates the paths of the irrigation passage 810 and the discharge collection passage 820, according to at least one illustrated implementation. The irrigation passage 810 connects the irrigation inlet port 813 located at the proximal end 803 of the cannula 105 with one or more irrigation outlet apertures 809 located proximate the distal end 805 of the cannula 105. The irrigation inlet port 813 may be substantially cylindrical, and sized and shaped to mate with the cleaning agent port 316 on the cannula coupler interface 203 when the cannula 105 is physically coupled with the cannula coupler interface 203. For example, the irrigation inlet port 813 may be a female connector or fastener, and the corresponding cleaning agent port 316 on the cannula coupler interface 203 may be a complementary male connector or fastener. The irrigation passage 810 may flatten as it progresses past the flanged portion 823 of the cannula 105 and goes towards the distal end 805 of the cannula. Such flattening may cause the irrigation passage 810 to form an arc shape that runs proximate the distal end 805 of the cannula 105 and extends between the various irrigation outlet apertures 809.

The discharge collection passage 820 provides a discharge flow path 819 between the discharge collection inlet port 817 and the discharge collection outlet port 821, and may be sized and shaped to mate with the vacuum port 318 on the cannula coupler interface 203 when the cannula 105 is physically coupled with the cannula coupler interface 203. For example, the discharge collection outlet port 821 may be a female connector or fastener, and the corresponding vacuum port 318 on the cannula coupler interface 203 may be a complementary male connector or fastener. The discharge collection passage 820 may have a smaller diameter at the distal end 805 of the cannula 105 compared to the diameter of the discharge collection passage 820 at the proximal end 803. Such narrowing may be gradual, such that the discharge collection passage 820 gradually tapers along the length of the cannula 105. Such narrowing may be abrupt and occur, for example, at a point where the discharge collection passage 820 moves out of the flanged portion 823 of the cannula. As discussed previously, a trap 833 may be located within the discharge collection passage 820, and sized, shaped, and oriented to capture particles of a specified size being carried within the discharge.

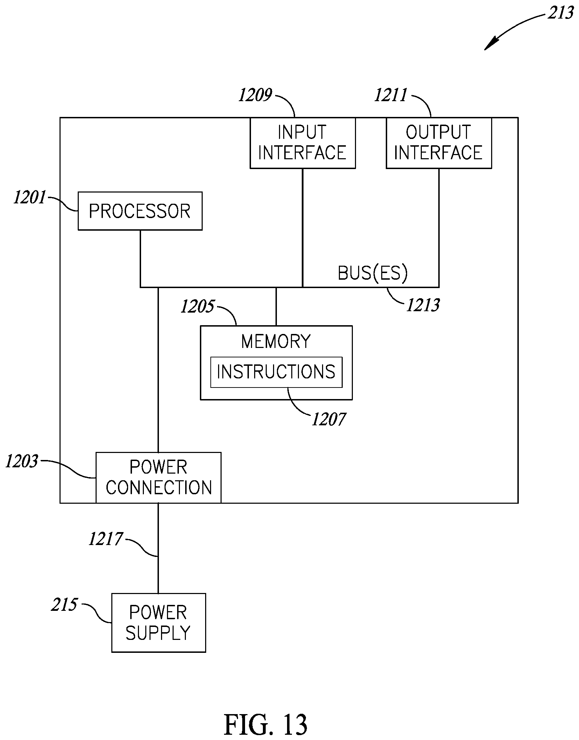

Figure 13 shows the controller circuitry 213 and the one or more power supplies 215, according to one illustrated implementation. The controller circuitry 213 includes at least one processor 1201, a connection 1203 to a power supply 215 (e.g., the one or more batteries), and one or more memories 1205 that store one or more sets of processor-executable instructions 1207, an input interface 1209 and an output interface 1211. Each of these components may be communicatively connected by bus(es) 1213, which can provide bidirectional communication between the various components of the controller circuitry 213. Bus(es) 1213 may take, for example, the form of a plurality of buses (*e.g*., data buses, instruction buses, power buses) included in at least one body.

The input interface 1209 may be electrically and communicatively coupled to the user-actable selectable controls 217 and be used to receive user inputs in the form of electrical signals. Such user inputs may include, for example, selecting between a plurality of irrigation programs stored as sets of processor-executable instructions 1207 by the one or more memories 1205. The output interface 1211 may be electrically and communicatively coupled to one or both of the pump assembly 207a and the vacuum assembly 207b. The output interface 1211 may be used to transmit electrical signals generated by the processor 1201 and that result in activating or deactivating the pump assembly 207a and/or vacuum assembly 207b.

The processor 1201 may be any logic processing unit, such as one or more central processing units (CPUs), digital signal processors (DSPs), application-specific integrated circuits (ASICs), field programmable gate arrays (FPGAs), etc. The power supply 215 may include one or more power supplies 215, which provide electrical power to the various components of the irrigation device 100 via power connections 1217. The power supplies 215 may be an internal power supply, such as a battery, energy source, fuel cell, or the like.

The one or more memories 1205 may include read-only memory ("ROM") and random access memory ("RAM"). The one or more memories 1205 may comprise a flash drive to store data and/or processor-executable instructions. In some implementations, the one or more memories 1205 may include a hard disk drive for reading from and writing to a hard disk, an optical disk drive for reading from and writing to removable optical disks, and/or a magnetic disk drive for reading from and writing to magnetic disks. The one or more memories 1205 may communicate with the processor 1201 via the system bus 1213. Those skilled in the relevant art will appreciate that other types of computer-readable media that can store data accessible by a computer may be employed, such as WORM drives, RAID drives, magnetic cassettes, flash memory cards, digital video disks ("DVD"), Bernoulli cartridges, RAMs, ROMs, smart cards, etc.

The one or more sets of processor-executable instructions 1207, when executed, cause the irrigation device 100 to perform one or more irrigation routines. Such irrigation routines, when executed, may cause the processor 1201 to transmit a first signal to the vacuum assembly 207b via the output interface 1211 at a specified time that results in the vacuum assembly 207b being turned on, thereby creating a vacuum force to cause discharge to move into the discharge collection inlet port 817 of the cannula 105 through the discharge fluidly communicative path 221 and into the discharge reservoir 205. Such irrigation routines, when executed, may cause the processor 1201 to transmit a second signal to the pump assembly 207a via the output interface 1211 at a specified time that results in the pump assembly 207a being turned on, thereby forcing cleaning agent to move from or exit the cleaning agent reservoir 201 through the cleaning fluid fluidly communicative path 219 to the cannula 105, where it exits through the irrigation outlet apertures 809 at pressure. The processor 1201 may activate the pump assembly 207a using a plurality of pulses in which the pump assembly 207a is activated and deactivated for short time durations (*e.g*., about two seconds) during the irrigation routine before the pump assembly 207a is turned off. The set of processor-executable instructions 1207 may provide for irrigation procedures of different time periods (*e.g*., up to 30 seconds, 30 seconds, one minute, or more than one minute).

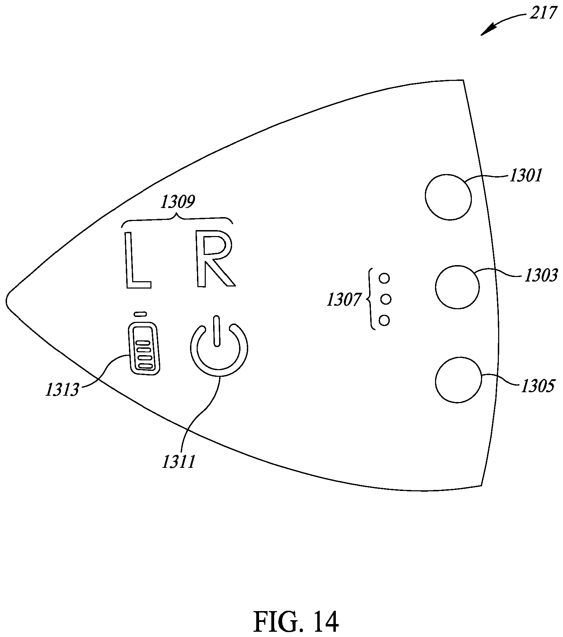

Figure 14 shows the user-actable selectable controls 217, according to at least one illustrated implementation. The user-actable selectable controls 217 include an ear selection button 1301 and a program selection button 1303, a start button 1305, a set of LEDs 1307, an ear activation indicator 1309, a power indicator 1311, and a charging indicator 1313. The ear selection button 1301 may be used to turn on the irrigation device 100 and select the ear to be cleaned. In some implementations, the ear selection may be performed by touching a touch-sensitive or touch-responsive (*e.g*., resistance, inductive or capacitance touch sensors) area of the irrigation device 100 over or proximate the desired ear (*e.g*., on or around junctions 135a and 135b). The program selection button 1303 may be used to select between a plurality of irrigation routines that may correspond to a plurality of sets of process-executable instructions 1207 stored within the one or more memories 1205. For example, the program selection button 1303 may be used to toggle or cycle through the plurality of irrigation routines. The set of LEDs 1307 may be used to show which routine is currently selected by the user. Thus, in some implementations, each LED may correspond to a location within the memory 1205 that stores instructions 1207 for a specific irrigation routine. The LED corresponding to the currently selected irrigation routine may be lit up. The start button 1305 may be used to start the currently selected irrigation routine.

In some implementations, the ear activation indicator 1309 may indicate which over-ear earpiece 101 is currently in operation, with the "R" corresponding to the over-ear earpiece 101a designed to fit over the user's right ear and the "L" corresponding to the over-ear earpiece 101 b designed to fit over the user's left ear. The power indicator 1311 indicates whether the irrigation device 100 is currently powered on and running a cleaning cycle. The charging indicator 1313 indicates whether the irrigation device 100 is currently electrically coupled to a power source such that the battery 215 is being charged. The amount of charge that the battery 215 currently holds may be directly related to the amount of the charging indicator 1313 that is lit up. In addition, or alternatively, a fully charged battery 215 may cause the charging indicator 1313 to switch from one color (e.g., red) that corresponds to the battery charging to a second color (*e.g*., green) that indicates that the battery is fully charged. Some implementations may include other signal and indicators. For example, one or more indicators may be used to signify that the pump assembly 207a is providing cleaning agent at too high of a pressure, that the vacuum assembly 207b is providing too strong of a vacuum, that the discharge passage 214 is blocked, *etc.* In some instances, the irrigation device 100 may automatically shutdown to prevent injury to the user or damage to the irrigation device 100. In some implementations, at least one of the user-actable selectable controls 217 may be used as a "kill" switch to automatically shut down the irrigation device 100.

In some implementations, the irrigation device 100 may include touch screen controls to control the operation of and provide feedback regarding the irrigation device 100. Such touch screen controls may be incorporated into a portion of the irrigation device 100. In some implementations, the irrigation device 100 may have wireless communication capabilities (*e.g*., via Bluetooth®, WiFi®, near field communications) such that the touch screen controls may be displayed via a wireless device, such as the user's smartphone/tablet or a dedicated tablet device).

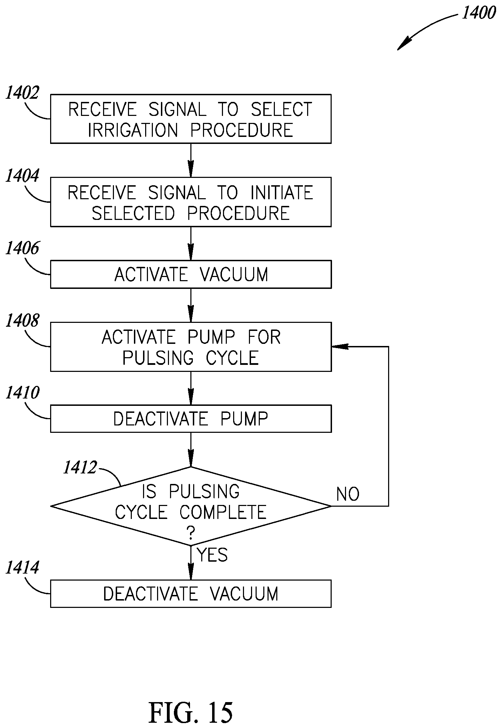

Figure 15 shows a method of operation 1400 of the irrigation device to perform an irrigation procedure, according to one illustrated implementation. At 1402, a signal is received that selects between the various irrigation procedures stored within the one or more memories 1205 of the controller circuitry 213.

At 1404, a signal is received that initiates the currently selected irrigation procedure.

At 1406, vacuum assembly 207b is activated via a signal generated by controller circuitry 213 and transmitted through the output interface 1211.

At 1408, the pump assembly 207a is activated for a pulsing cycle. As noted before, activating the pump assembly 207a results in cleaning agent being dispensed from the cleaning agent reservoir 201 via the cleaning fluid fluidly communicative path 219 towards the cannula 105, where the cleaning agent exits under pressure. Thus, sending the signal to activate the pump assembly 207a causes a certain quantity of cleaning agent to be dispensed from the cleaning agent reservoir 201.

At 1410, the pump assembly 207a is deactivated after a time-delay is observed, thus providing one pulse of cleaning agent. The time delay and resulting pulse duration may be up to 2 seconds, for example. In some implementations, the time delay may be more or less than 2 seconds.

At 1412, a determination is made if the pulse cycle is complete. If the pulse cycle is not complete, then the operation 1400 goes to 1408 to activate the pump assembly 207a. In some implementations, each successive pulse may serve to soften and dislodge ear wax within the user's auditory canal. Each pulse cycle may be, for example, up to 30 seconds, one minute, 90 seconds, or longer. If the pulse cycle is complete, then the operation 1400 proceeds to 1414.

At 1414, the vacuum assembly 207b is deactivated to end the irrigation procedure.

Various implementations can be made without departing from the scope of the appended claims.

## Claims

1. A device (100) to irrigate ear canals of human ears, the device (100) comprising:
a cannula (105a) including:
a body that tapers from a proximal end of the cannula (105a) toward the distal end of the cannula (105a), the body positionable within one human ear such that body impacts a side wall of an auditory canal of the ear before the distal end of the cannula (105a) impacts an ear drum of the ear;
an irrigation inlet port (813) positioned at the proximal end of the cannula (105a);
a discharge collection outlet port (821) positioned at the proximal end of the cannula (105a);
a plurality of irrigation outlet apertures (809) positioned relatively toward the distal end of the cannula (105a) with respect to the irrigation inlet port (813), the irrigation outlet apertures (809) in fluid communication with the irrigation inlet port (813); and
a discharge collection inlet port (817) positioned relatively toward the distal end of the cannula (105a) with respect to the discharge collection outlet port (821), the discharge collection inlet port (817) in fluid communication with the discharge collection outlet port (821); and
an over-ear earpiece sized and dimensioned to be worn over human ears and having a cavity sized and dimensioned to receive one human ear, the over-ear earpiece including:
a cannula coupler interface (203) to which the cannula (105a) is selectively detachably physically coupleable;
a cleaning fluid fluidly communicative path (219) configured to provide fluid communication between a cleaning agent reservoir (201) and the cannula coupler interface (203) such that cleaning agent contained within a cleaning agent reservoir (201) can exit a cleaning agent reservoir (201) and proceed through the cleaning fluid fluidly communicative path (219) to the cannula (105a) when the cannula (105a) is coupled to the cannula coupler interface (203);
a discharge fluidly communicative path (221) configured to provide fluid communication between a discharge collection reservoir (205) and the cannula coupler interface (203) such that discharge from an ear can exit the cannula (105a) to the discharge fluidly communicative path (221) to a discharge collection reservoir (205) when the cannula (105a) is coupled to the cannula coupler interface (203); and
a housing (210) including a lid (209) and a pan (211), the lid (209) including one or more coupling features usable to secure a cleaning agent reservoir (201) and a discharge collection reservoir (205) to the lid (209).

2. The device of claim 1, wherein the over-ear earpiece is a first over-ear earpiece, the cannula coupler interface is a first cannula coupler interface, the cleaning fluid fluidly communicative path is a first cleaning fluid fluidly communicative path, the discharge fluidly communicative path is a first discharge fluidly communicative path, the cannula is a first cannula, the device further comprising:
a second cannula including a body that tapers from a proximal end of the second cannula toward the distal end of the second cannula, an irrigation inlet port positioned at the proximal end of the cannula, and a discharge collection outlet port positioned at the proximal end of the cannula; and
a second over-ear earpiece sized and dimensioned to be worn over human ears and having a cavity sized and dimensioned to receive one human ear, the second over-ear earpiece comprising:
a second cannula coupler interface to which the second cannula is selectively detachably physically coupleable;
a second cleaning fluid fluidly communicative path configured to provide fluid communication between a cleaning agent reservoir and the second cannula coupler interface; and
a second discharge fluidly communicative path configured to provide fluid communication between a discharge collection reservoir and the second cannula coupler interface.

3. The device of claim 1, wherein:
the cleaning fluid fluidly communicative path includes a cleaning agent port that mates with the irrigation inlet port of the cannula when the cannula is coupled to the cannula coupler interface; and
the discharge fluidly communication path includes a vacuum port that mates with the discharge collection outlet port of the cannula when the cannula is coupled to the cannula coupler interface.

4. The device of claim 1, wherein the over-ear earpiece further comprises:
a resilient or conformable annular membrane which at least partially forms the cavity sized and dimensioned to receive an ear.

5. The device of claim 1, wherein the over-ear earpiece further comprises:
a cleaning agent reservoir to hold cleaning agent; and
a discharge collection reservoir to collect discharge from an ear.

6. The device of claim 1, wherein the over-ear earpiece further comprises:
at least one pump assembly fluidly coupled to move cleaning agent along the cleaning fluid fluidly communicative path from a cleaning agent reservoir toward the cannula coupler interface.

7. The device of claim 1, wherein the over-ear earpiece further comprises:
at least one pump assembly fluidly coupled to apply a negative pressure to move discharge from an ear along the discharge fluidly communicative path from the cannula coupler interface toward a discharge collection reservoir.

8. The device of any of claims 6 and 7, wherein the over-ear earpiece further comprises:
set of controller circuitry housed in the housing and communicatively coupled to control the at least one pump assembly.

9. The device of claim 8, wherein the over-ear earpiece further comprises:
a number of user-actable selectable controls accessible from an exterior of the device, the user-actable selectable controls communicatively coupled to the set of controller circuitry communicatively coupled to control the at least one pump assembly.

10. The device of claim 1, wherein the cannula further comprises a flow path that extends between the discharge collection inlet port and the discharge collection outlet port and a trap positioned in the flow path between the discharge collection inlet port and the discharge collection outlet port, the trap configured to trap physical debris while passing at least one of a quantity of a liquid and air.

11. The device of any of claims 6 or 7, further comprising:
a processor, and
a nontransitory computer-readable medium communicatively coupled to the processor, wherein the nontransitory computer-readable medium stores processor-executable instructions that specifically program the processor to:
provide a number of signals to the at least one pump assembly that causes the at least one pump assembly to dispense a first quantity of cleaning agent along the cleaning fluid fluidly communicative path from a cleaning agent reservoir and to produce the negative pressure along at least a portion of the discharge fluidly communicative path.

12. The device of claim 1, wherein the cannula includes a beveled portion at the distal end of the cannula, wherein a cross-sectional area of the distal end of the cannula is less than a cross- sectional area of the proximal end of the cannula.

13. The device of claim 1, wherein the cannula coupler interface includes an annular wall configured to physically engage a corresponding interface of a cannula.

## Patentansprüche

1. Vorrichtung (100) zum Spülen von Ohrkanälen von menschlichen Ohren, wobei die Vorrichtung (100) Folgendes umfasst:
eine Kanüle (105a), die Folgendes umfasst:
einen Körper, der von einem proximalen Ende der Kanüle (105a) in Richtung des distalen Endes der Kanüle (105a) verjüngt ist, wobei der Körper derart in einem menschlichen Ohr positionierbar ist, dass der Körper auf eine Seitenwand eines Gehörgangs des Ohrs auftrifft, bevor das distale Ende der Kanüle (105a) auf ein Trommelfell des Ohrs auftrifft;
eine Spülungseinlassöffnung (813), die an dem proximalen Ende der Kanüle (105a) positioniert ist;
eine Auslaufsammlungs-Auslassöffnung (821), die an dem proximalen Ende der Kanüle (105a) positioniert ist;
eine Vielzahl von Spülungsmündungen (809), die in Bezug auf die Spülungseinlassöffnung (813) relativ in Richtung des distalen Endes der Kanüle (105a) positioniert sind, wobei die Spülungsmündungen (809) mit der Spülungseinlassöffnung (813) in Fluidverbindung stehen; und
eine Auslaufsammlungs-Einlassöffnung (817), die in Bezug auf die Auslaufsammlungs-Auslassöffnung (821) relativ in Richtung des distalen Endes der Kanüle (105a) positioniert ist, wobei die Auslaufsammlungs-Einlassöffnung (817) mit der Auslaufsammlungs-Auslassöffnung (821) in Fluidverbindung steht; und
ein Über-Ohr-Ohrstück, das dazu bemessen und dimensioniert ist, über menschlichen Ohren getragen zu werden und eine Höhlung aufweist, die dazu bemessen und dimensioniert ist, ein menschliches Ohr aufzunehmen, wobei das Über-Ohr-Ohrstück Folgendes umfasst:
einen Kanülenkopplungsanschluss (203), an den die Kanüle (105a) selektiv abtrennbar physisch gekoppelt werden kann;
einen fluidisch kommunizierenden Reinigungsfluidweg (219), der dazu konfiguriert ist, eine Fluidverbindung zwischen einem Reinigungsmittelbehälter (201) und dem Kanülenkopplungsanschluss (203) bereitzustellen, sodass in einem Reinigungsmittelbehälter (201) enthaltendes Reinigungsmittel einen Reinigungsmittelbehälter (201) verlassen kann und sich durch den fluidisch kommunizierenden Reinigungsfluidweg (219) zu der Kanüle (105a) voranbewegen kann, wenn die Kanüle (105a) an den Kanülenkopplungsanschluss (203) gekoppelt ist;
einen fluidisch kommunizierenden Auslaufweg (221), der dazu konfiguriert ist, eine Fluidverbindung zwischen einem Auslaufsammelbehälter (205) und dem Kanülenkopplungsanschluss (203) bereitzustellen, sodass Auslauf aus einem Ohr die Kanüle (105a) zu dem fluidisch kommunizierenden Auslaufweg (221) zu einem Auslaufsammelbehälter (205) verlassen kann, wenn die Kanüle (105a) an den Kanülenkopplungsanschluss (203) gekoppelt ist; und
ein Gehäuse (210), umfassend einen Deckel (209) und eine Schale (211), wobei der Deckel (209) ein oder mehrere Kopplungsmerkmale umfasst, die verwendet werden können, um einen Reinigungsmittelbehälter (201) und einen Auslaufsammelbehälter (205) an dem Deckel (209) zu befestigen.

2. Vorrichtung nach Anspruch 1, wobei es sich bei dem Über-Ohr-Ohrstück um ein erstes Über-Ohr-Ohrstück handelt, es sich bei dem Kanülenkopplungsanschluss um einen ersten Kanülenkopplungsanschluss handelt, es sich bei dem fluidisch kommunizierenden Reinigungsfluidweg um einen ersten fluidisch kommunizierenden Reinigungsfluidweg handelt, es sich bei dem fluidisch kommunizierenden Auslaufweg um einen ersten fluidisch kommunizierenden Auslaufweg handelt, es sich bei der Kanüle um eine erste Kanüle handelt und die Vorrichtung ferner Folgendes umfasst:
eine zweite Kanüle, umfassend einen Körper, der von einem proximalen Ende der zweiten Kanüle in Richtung des distalen Endes der zweiten Kanüle verjüngt ist, eine Spülungseinlassöffnung, die an dem proximalen Ende der Kanüle positioniert ist und eine Auslaufsammlungs-Auslauföffnung, die an dem proximalen Ende der Kanüle positioniert ist; und
ein zweites Über-Ohr-Ohrstück, das dazu bemessen und dimensioniert ist, über menschlichen Ohren getragen zu werden und eine Höhlung aufweist, die dazu bemessen und dimensioniert ist, ein menschliches Ohr aufzunehmen, wobei das zweite Über-Ohr-Ohrstück Folgendes umfasst:
einen zweiten Kanülenkopplungsanschluss, an den die zweite Kanüle selektiv abtrennbar physisch gekoppelt werden kann;
einen zweiten fluidisch kommunizierenden Reinigungsfluidweg, der dazu konfiguriert ist, eine Fluidverbindung zwischen einem Reinigungsmittelbehälter und dem zweiten Kanülenkopplungsanschluss bereitzustellen; und
einen zweiten fluidisch kommunizierenden Auslaufweg, der dazu konfiguriert ist, eine Fluidverbindung zwischen einem Auslaufsammelbehälter und dem zweiten Kanülenkopplungsanschluss bereitzustellen.

3. Vorrichtung nach Anspruch 1, wobei:
der fluidisch kommunizierenden Reinigungsfluidweg eine Reinigungsmittelöffnung umfasst, die mit der Spülungseinlassöffnung der Kanüle zusammenpasst, wenn die Kanüle an den Kanülenkopplungsanschluss gekoppelt ist; und
der fluidisch kommunizierenden Auslaufweg eine Vakuumöffnung umfasst, die mit der Auslaufsammlungs-Auslassöffnung der Kanüle zusammenpasst, wenn die Kanüle an den Kanülenkopplungsanschluss gekoppelt ist.

4. Vorrichtung nach Anspruch 1, wobei das Über-Ohr-Ohrstück ferner Folgendes umfasst:
eine elastische oder anpassungsfähige ringförmige Membran, die mindestens teilweise die zum Aufnehmen eines Ohrs bemessene und dimensionierte Höhlung bildet.

5. Vorrichtung nach Anspruch 1, wobei das Über-Ohr-Ohrstück ferner Folgendes umfasst:
einen Reinigungsmittelbehälter zum Halten von Reinigungsmittel; und
einen Auslaufsammelbehälter zum Sammeln von Auslauf aus einem Ohr.

6. Vorrichtung nach Anspruch 1, wobei das Über-Ohr-Ohrstück ferner Folgendes umfasst:
mindestens eine Pumpenanordnung, die fluidisch gekoppelt ist, um Reinigungsmittel entlang des fluidisch kommunizierenden Reinigungsfluidwegs von einem Reinigungsmittelbehälter in Richtung des Kanülenkopplungsanschlusses zu bewegen.

7. Vorrichtung nach Anspruch 1, wobei das Über-Ohr-Ohrstück ferner Folgendes umfasst:
mindestens eine Pumpenanordnung, die fluidisch gekoppelt ist, um einen Unterdruck anzulegen, um Auslauf aus einem Ohr entlang des fluidisch kommunizierenden Auslaufwegs von dem Kanülenkopplungsanschluss in Richtung eines Auslaufsammelbehälters zu bewegen.

8. Vorrichtung nach einem der Ansprüche 6 und 7, wobei das Über-Ohr-Ohrstück ferner Folgendes umfasst:
einen Satz von Steuerkreisen, die in dem Gehäuse untergebracht sind und kommunizierend gekoppelt sind, um die mindestens eine Pumpenanordnung zu steuern.

9. Vorrichtung nach Anspruch 8, wobei das Über-Ohr-Ohrstück ferner Folgendes umfasst:
eine Anzahl von benutzerbetätigbaren auswählbaren Bedienungselementen, die von einem Äußeren der Vorrichtung aus zugänglich sind, wobei die benutzerbetätigbaren auswählbaren Bedienungselemente kommunizierend an den Satz von Steuerkreisen gekoppelt sind, die kommunizierend gekoppelt sind, um die mindestens eine Pumpenanordnung zu steuern.

10. Vorrichtung nach Anspruch 1, wobei die Kanüle ferner Folgendes umfasst: einen Strömungsweg, der sich zwischen der Auslaufsammlungs-Einlassöffnung und der Auslaufsammlungs-Auslassöffnung erstreckt, und einen in dem Strömungsweg zwischen der Auslaufsammlungs-Einlassöffnung und der Auslaufsammlungs-Auslassöffnung positionierten Abscheider, wobei der Abscheider dazu konfiguriert ist, physischen Schmutz abzuscheiden, während er eine Menge einer Flüssigkeit und/oder Luft durchlässt.

11. Vorrichtung nach einem der Ansprüche 6 und 7, die ferner Folgendes umfasst:
einen Prozessor, und
ein nichtflüchtiges computerlesbares Medium, das kommunizierend an den Prozessor gekoppelt ist wobei das nichtflüchtige computerlesbare Medium prozessorausführbare Anweisungen speichert, die den Prozessor spezifisch dazu programmieren:
der mindestens einen Pumpenanordnung eine Anzahl von Signalen bereitzustellen, die die mindestens eine Pumpenanordnung veranlassen, eine erste Menge von Reinigungsmittel entlang des fluidisch kommunizierenden Reinigungsfluidwegs aus einem Reinigungsmittelbehälter auszugeben und den Unterdruck entlang mindestens eines Abschnitts des fluidisch kommunizierenden Auslaufwegs zu erzeugen.

12. Vorrichtung nach Anspruch 1, wobei die Kanüle einen abgeschrägten Abschnitt an dem distalen Ende der Kanüle umfasst, wobei eine Querschnittsfläche des distalen Endes der Kanüle kleiner ist als eine Querschnittsfläche des proximalen Endes der Kanüle.

13. Vorrichtung nach Anspruch 1, wobei der Kanülenkopplungsanschluss eine ringförmige Wand umfasst, die für den physischen Eingriff mit einem entsprechenden Anschluss einer Kanüle konfiguriert ist.

## Revendications

1. Dispositif (100) pour irriguer les canaux auriculaires d'oreilles humaines, le dispositif (100) comprenant :
une canule (105a) comportant :
un corps qui rétrécit depuis une extrémité proximale de la canule (105a) en direction de l'extrémité distale de la canule (105a), le corps pouvant être positionné à l'intérieur d'une oreille humaine de telle façon que le corps touche une paroi latérale d'un conduit auditif de l'oreille avant que l'extrémité distale de la canule (105a) touche un tympan de l'oreille ;
un orifice d'entrée d'irrigation (813) positionné à l'extrémité proximale de la canule (105a) ;
un orifice de sortie de collecte d'évacuation (821) positionné à l'extrémité proximale de la canule (105a) ;
une pluralité d'ouvertures de sortie d'irrigation (809) positionnées relativement en direction de l'extrémité distale de la canule (105a) par rapport à l'orifice d'entrée d'irrigation (813), les ouvertures de sortie d'irrigation (809) étant en communication fluidique avec l'orifice d'entrée d'irrigation (813) ; et
un orifice d'entrée de collecte d'évacuation (817) positionné relativement en direction de l'extrémité distale de la canule (105a) par rapport à l'orifice de sortie de collecte d'évacuation (821), l'orifice d'entrée de collecte d'évacuation (817) étant en communication fluidique avec l'orifice de sortie de collecte d'évacuation (821) ; et
une coque supra-auriculaire conçue et dimensionnée pour être portée par-dessus des oreilles humaines et ayant une cavité conçue et dimensionnée pour recevoir une oreille humaine, la coque supra-auriculaire comportant :
une interface d'accouplement de canule (203) à laquelle la canule (105a) peut être sélectivement couplée physiquement de manière détachable ;
un chemin de fluide de nettoyage à communication fluidique (219) configuré pour assurer une communication fluidique entre un réservoir d'agent de nettoyage (201) et l'interface d'accouplement de canule (203) de telle façon que l'agent de nettoyage contenu à l'intérieur d'un réservoir d'agent de nettoyage (201) peut sortir d'un réservoir d'agent de nettoyage (201) et passer dans la canule (105a) par le biais du chemin de fluide de nettoyage à communication fluidique (219) lorsque la canule (105a) est couplée à l'interface d'accouplement de canule (203) ;
un chemin d'évacuation à communication fluidique (221) configuré pour assurer une communication fluidique entre un réservoir de collecte d'évacuation (205) et l'interface d'accouplement de canule (203) de telle façon que l'évacuation venant d'une oreille peut sortir de la canule (105a) vers le chemin d'évacuation à communication fluidique (221) jusqu'à un réservoir de collecte d'évacuation (205) lorsque la canule (105a) est couplée à l'interface d'accouplement de canule (203) ; et
un boîtier (210) comportant un couvercle (209) et une cuvette (211), le couvercle (209) comportant un ou plusieurs éléments de couplage utilisables pour fixer un réservoir d'agent de nettoyage (201) et un réservoir de collecte d'évacuation (205) sur le couvercle (209).

2. Dispositif selon la revendication 1, dans lequel la coque supra-auriculaire est une première coque supra-auriculaire, l'interface d'accouplement de canule est une première interface d'accouplement de canule, le chemin de fluide de nettoyage à communication fluidique est un premier chemin de fluide de nettoyage à communication fluidique, le chemin d'évacuation à communication fluidique est un premier chemin d'évacuation à communication fluidique, la canule est une première canule, le dispositif comprenant en outre :
une deuxième canule comportant un corps qui rétrécit depuis une extrémité proximale de la deuxième canule en direction de l'extrémité distale de la deuxième canule, un orifice d'entrée d'irrigation positionné à l'extrémité proximale de la canule, et un orifice de sortie de collecte d'évacuation positionné à l'extrémité proximale de la canule ; et
une deuxième coque supra-auriculaire conçue et dimensionnée pour être portée par-dessus des oreilles humaines et ayant une cavité conçue et dimensionnée pour recevoir une oreille humaine, la deuxième coque supra-auriculaire comprenant :
une deuxième interface d'accouplement de canule à laquelle la deuxième canule peut être sélectivement couplée physiquement de manière détachable ;
un deuxième chemin de fluide de nettoyage à communication fluidique configuré pour assurer une communication fluidique entre un réservoir d'agent de nettoyage et la deuxième interface d'accouplement de canule ; et
un deuxième chemin d'évacuation à communication fluidique configuré pour assurer une communication fluidique entre un réservoir de collecte d'évacuation et la deuxième interface d'accouplement de canule.

3. Dispositif selon la revendication 1, dans lequel :
le chemin de fluide de nettoyage à communication fluidique comporte un orifice d'agent de nettoyage qui s'accouple à l'orifice d'entrée d'irrigation de la canule lorsque la canule est couplée à l'interface d'accouplement de canule ; et
le chemin d'évacuation à communication fluidique comporte un orifice de vide qui s'accouple à l'orifice de sortie de collecte d'évacuation de la canule lorsque la canule est couplée à l'interface d'accouplement de canule.

4. Dispositif selon la revendication 1, dans lequel la coque supra-auriculaire comprend en outre :
une membrane annulaire élastique ou conformable qui forme au moins partiellement la cavité conçue et dimensionnée pour recevoir une oreille.

5. Dispositif selon la revendication 1, dans lequel la coque supra-auriculaire comprend en outre :
un réservoir d'agent de nettoyage pour contenir de l'agent de nettoyage ; et
un réservoir de collecte d'évacuation pour collecter l'évacuation d'une oreille.

6. Dispositif selon la revendication 1, dans lequel la coque supra-auriculaire comprend en outre :
au moins un ensemble pompe couplé fluidiquement pour déplacer de l'agent de nettoyage le long du chemin de fluide de nettoyage à communication fluidique depuis un réservoir d'agent de nettoyage jusqu'à l'interface d'accouplement de canule.

7. Dispositif selon la revendication 1, dans lequel la coque supra-auriculaire comprend en outre :
au moins un ensemble pompe couplé fluidiquement pour appliquer une pression négative afin de déplacer l'évacuation d'une oreille le long du chemin d'évacuation à communication fluidique depuis l'interface d'accouplement de canule vers un réservoir de collecte d'évacuation.

8. Dispositif selon l'une quelconque des revendications 6 et 7, dans lequel la coque supra-auriculaire comprend en outre :
un ensemble de circuiterie de commande logé dans le boîtier et couplé communicativement pour commander ledit au moins un ensemble pompe.

9. Dispositif selon la revendication 8, dans lequel la coque supra-auriculaire comprend en outre :
un certain nombre de commandes sélectionnables actionnables par un utilisateur, accessibles depuis un extérieur du dispositif, les commandes sélectionnables actionnables par un utilisateur étant couplées communicativement à l'ensemble de circuiterie de commande couplé communicativement pour commander ledit au moins un ensemble pompe.

10. Dispositif selon la revendication 1, dans lequel la canule comprend en outre un chemin d'écoulement qui s'étend entre l'orifice d'entrée de collecte d'évacuation et l'orifice de sortie de collecte d'évacuation, et un piège positionné dans le chemin d'écoulement entre l'orifice d'entrée de collecte d'évacuation et l'orifice de sortie de collecte d'évacuation, le piège étant configuré pour piéger des débris physiques tout en laissant passer l'un au moins parmi une quantité d'un liquide et de l'air.

11. Dispositif selon l'une quelconque des revendications 6 ou 7, comprenant en outre :
un processeur, et
un support non transitoire lisible par ordinateur couplé communicativement au processeur, dans lequel le support non transitoire lisible par ordinateur stocke des instructions exécutables par le processeur qui programment spécifiquement le processeur pour :
fournir audit au moins un ensemble pompe un certain nombre de signaux qui amènent ledit au moins un ensemble pompe à délivrer une première quantité d'agent de nettoyage le long du chemin de fluide de nettoyage à communication fluidique depuis un réservoir d'agent de nettoyage et à produire la pression négative le long d'au moins une partie du chemin d'évacuation à communication fluidique.

12. Dispositif selon la revendication 1, dans lequel la canule comporte une partie biseautée à l'extrémité distale de la canule, dans lequel une aire de section transversale de l'extrémité distale de la canule est inférieure à une aire de section transversale de l'extrémité proximale de la canule.

13. Dispositif selon la revendication 1, dans lequel l'interface d'accouplement de canule comporte une paroi annulaire configurée pour venir physiquement en prise avec une interface correspondante d'une canule.
